(19) Europäisches Patentamt / European Patent Office / Office européen des brevets

(11) **EP 4 482 790 B1**

(12) **EUROPÄISCHE PATENTSCHRIFT**

(45) Veröffentlichungstag und Bekanntmachung des Hinweises auf die Patenterteilung:
**13.08.2025 Patentblatt 2025/33**

(21) Anmeldenummer: **23707356.4**

(22) Anmeldetag: **24.02.2023**

(51) Internationale Patentklassifikation (IPC):
**C01B 32/50** (2017.01)    **C09C 1/48** (2006.01)
**C09C 1/50** (2006.01)    **C12P 5/02** (2006.01)
**C10L 3/10** (2006.01)    **C01B 32/05** (2017.01)
**C01B 3/24** (2006.01)    **C10L 3/08** (2006.01)
**C10L 1/04** (2006.01)    **C10G 2/00** (2006.01)

(52) Gemeinsame Patentklassifikation (CPC):
**C10L 3/08; C01B 3/24; C01B 32/50; C09C 1/48;**
**C09C 1/50; C10G 2/50; C10L 3/104; C12P 5/023;**
C10L 2290/26; C10L 2290/542; C10L 2290/543;
C10L 2290/548

(86) Internationale Anmeldenummer:
**PCT/EP2023/054656**

(87) Internationale Veröffentlichungsnummer:
**WO 2023/161404 (31.08.2023 Gazette 2023/35)**

(54) **VERFAHREN ZUR ENTFERNUNG UND IMMOBILISIERUNG VON KOHLENSTOFFDIOXID AUS DER ATMOSPHÄRE UND/ODER EINEM ABGAS**

METHOD FOR REMOVING AND IMMOBILIZING CARBON DIOXIDE FROM THE ATMOSPHERE AND / OR AN EXHAUST GAS

PROCÉDÉ D'ÉLIMINATION ET D'IMMOBILISATION DU DIOXYDE DE CARBONE DE L'ATMOSPHÈRE ET/OU D'UN GAZ D'ÉCHAPPEMENT

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **25.02.2022 EP 22158779**

(43) Veröffentlichungstag der Anmeldung:
**01.01.2025 Patentblatt 2025/01**

(73) Patentinhaber: **FLD Technologies GmbH**
**63071 Offenbach am Main (DE)**

(72) Erfinder: **FULDE, Marek**
**63071 Offenbach (DE)**

(74) Vertreter: **Kudla, Karsten**
**Patentanwälte**
**Isenbruck Bösl Hörschler PartG mbB**
**Eastsite One**
**Seckenheimer Landstrasse 4**
**68163 Mannheim (DE)**

(56) Entgegenhaltungen:
WO-A1-2018/112654    CN-A- 107 058 055
GB-A- 2 470 452    US-A1- 2012 276 616
US-A1- 2021 371 277

- MURADOV N Z ET AL: ""Green" path from fossil-based to hydrogen economy: An overview of carbon-neutral technologies", INTERNATIONAL JOURNAL OF HYDROGEN ENERGY, ELSEVIER, AMSTERDAM, NL, vol. 33, no. 23, 1 December 2008 (2008-12-01), pages 6804 - 6839, XP025691175, ISSN: 0360-3199, [retrieved on 20081028], DOI: 10.1016/ J.IJHYDENE.2008.08.054
- STEINBERG M ED - KURT EROL ET AL: "Fossil fuel decarbonization technology for mitigating global warming", INTERNATIONAL JOURNAL OF HYDROGEN ENERGY, ELSEVIER, AMSTERDAM, NL, vol. 24, no. 8, 1 August 1999 (1999-08-01), pages 771 - 777, XP004173561, ISSN: 0360-3199, DOI: 10.1016/S0360-3199(98)00128-1

**Beschreibung**

Die Erfindung betrifft ein Verfahren zur Entfernung und Immobilisierung von Kohlenstoffdioxid ($CO_2$) aus der Atmosphäre und/oder einem Abgas. Hierbei wird $CO_2$ aus der Luft und/oder einem Abgas aufgenommen und in festen Kohlenstoff umgewandelt, der dauerhaft gelagert wird.

Zur Reduktion von $CO_2$ in der Atmosphäre sind dem Fachmann sogenannte "carbon dioxide capture and storage" (CCS) -Verfahren bekannt. Der Treibhauseffekt ist seit etwa 200 Jahren bekannt (J.B. Fourier), er wurde auf der ersten Weltklimakonferenz 1979 in Genf diskutiert. Die Entfernung aus der Atmosphäre und Immobilisierung von Treibhausgasen, insbesondere $CO_2$, wird auch als Sequestrierung bezeichnet und umfasst die Abscheidung von $CO_2$ aus industriellen Quellen und in Kraftwerken, den Transport zu einem Lagerort und schließlich eine dauerhafte Einlagerung von $CO_2$, wobei dieses von der Atmosphäre isoliert wird.

Zur Vermeidung von $CO_2$-Emissionen wird bei bekannten CCS-Verfahren $CO_2$ abgeschieden und das Gas anschließend gelagert. Bei der Anwendung dieses Verfahrens wird zu behandelndes Abgas zuerst von Partikeln und Schwefelverbindungen befreit. Im darauffolgenden Schritt wird $CO_2$ aus dem Abgas abgetrennt, komprimiert, gegebenenfalls abtransportiert und unterirdisch gelagert. Es ist umstritten, ob dabei eine dauerhafte Bindung von $CO_2$ an Mineralien stattfindet oder mit erneuter Freisetzung des Gases in die Atmosphäre zu rechnen ist. Weiterhin besteht das Risiko, dass das unterirdische Verpressen von $CO_2$ unter hohem Druck zu Erdbeben führen könnte. Darüber hinaus könnte eine Vermischung mit und ein Lösen von $CO_2$ im Grundwasser sogenannte "Kalt-Geysire" hervorrufen, die ebenfalls erneute Emission verursachen würden.

Ein weiterer Nachteil der CCS-Technologie liegt in fehlender Infrastruktur für den erforderlichen Transport von $CO_2$. Emissionsquellen wie Kraftwerke oder Zementwerke liegen in der Regel weit von geeigneten $CO_2$-Lagerorten entfernt, was den Bau von geeigneten Rohrleitungen erforderlich machen würde.

In DE 10 2013 112 205 A1 wird hingegen darauf hingewiesen, dass Kohlenstoff in fester Form zum Beispiel in alten Kohlenflözen langfristig und problemlos eingelagert werden kann.

Kamman et al., in "Biokohle: Ein Weg zur dauerhaften Kohlenstoff-Sequestrierung", Umweltbeobachtungs- und Klimafolgenforschungsstation Linden, Hessisches Landesamt für Umwelt und Geologie, Institut für Pflanzenökologie der Justus-Liebig-Universität Gießen, April 2010, Seiten 1 bis 8, DOI 14902790298116661323 diskutieren die Verwendung von Biokohle in Böden.

DE 10 2007 037 672 A1 beschreibt ein Verfahren zur Harmonisierung von Strom-Angebots/-Verbrauchs-Verläufen durch Zwischenspeicher und Einbeziehung einer $CO_2$-Verwertung. Aus $CO_2$-haltigem Abgas wird reines $CO_2$ gewonnen und $CO_2$ zwischengelagert oder endgelagert, was einem bekannten CCS-Verfahren entspricht. Die Spaltung von Kohlenwasserstoffen ist nicht vorgesehen und eine Lagerung von Kohlenstoff in fester Form findet nicht statt.

WO 2015/044407 A1 hat ein Verfahren zur Speicherung von Strom aus erneuerbaren Quellen zum Gegenstand. Dabei wird reiner Wasserstoff, der durch Elektrolyse von Wasser gewonnen wird, mit reinem $CO_2$ oder einem $CO_2/CO/H_2$-Gemisch zu Methan umgesetzt. Methan wird zwischengelagert und anschließend zu Kohlenstoff und Wasserstoff gespalten. Der Kohlenstoff wird zur Herstellung von $CO_2$ oder eines $CO_2/CO/H_2$-Gemisches wiederverwendet. $CO_2$ wird nicht gelagert oder aus der Atmosphäre entfernt, da der Kohlenstoff in dem Verfahren vollständig im Kreislauf geführt wird.

J. Temple in "Klima-Ingenieure machen Ernst, CO2 aus der Luft waschen", Technology Review, Seiten 49 bis 52, Juni 2017, beschreibt das Auswaschen von $CO_2$ aus der Luft zur Pflanzen-Düngung. Zur Abtrennung werden mit Adsorptionsmittel beschichtete Filter eingesetzt, die zur Desorption des $CO_2$ mit entsprechendem Energiebedarf erwärmt werden müssen.

WO 2014/170184 A1 betrifft eine Adsorptionsvorrichtung zur Abtrennung von Gas, insbesondere für $CO_2$, ebenso wie WO 2015/185434 A1.

WO 2016/005226 A1 ist auf ein Verfahren zur zyklischen Adsorption beziehungsweise Desorption in CCS-Verfahren gerichtet.

Wurzbacher, J. A., in "Development of a temperature-vacuum swing process for CO2 capture from ambient air", 2015, ETH Zürich Research Collection, https://doi.org/10.3929/ethz-a-010432423, beschreibt ein Verfahren zur Extraktion von konzentriertem $CO_2$ aus der Atmosphäre mittels Adsorption.

Gebald, C., in "Development of amine-functionalized adsorbent for carbon dioxide capture from atmospheric air", 2014, ETH Zürich Research Collection, https://doi.org/10.3929/ethza-010171623, offenbart die Verwendung von Aminosilanen als Adsorbentien, die zur Bindung von $CO_2$ aus der Atmosphäre eingesetzt werden.

Auch die Schriften US 9,095,813, US 2015/14815661, US 8,119,091, US 8,728,428, US 9,975,100, US 8,871,008, US 9,637,393, US 2017/15622883, US 2017/ 15591324, CA 2017051581 sind auf die Entfernung von $CO_2$ aus der Atmosphäre gerichtet.

E. Katifu, in "Carbon dioxide absorption using fresh water algae and identifying potential uses of algal biomass", Faculty of Engineering and the Built Environment, University of the Witwatersrand, 2011, Johannesburg, behandelt den Einsatz von Mikro-Algen zur Reduktion von $CO_2$-Emissionen.

Die Dokumente GB 2 470 452 A, CN 107 058 055 A und US 2012/276616 A befassen sich mit der Herstellung von Biomethan aus pflanzlicher Biomasse. Aus den Schriften MURADOV N Z ET AL: "Green path from fossil-based to hydrogen economy: An overview of carbon-neutral technologies", INTERNATIONAL JOURNAL OF HYDROGEN ENERGY, ELSEVIER, AMSTERDAM, NL, Bd. 33, Nr. 23, (2008-12-01), Seiten 6804-6839, DOI: 10.1016/ J.IJHYDENE.2008.08.054; US 2021/371277 A; und STEINBERG M ED - KURT EROL ET AL: "Fossil fuel decarbonization technology for mitigating global warming", INTERNATIONAL JOURNAL OF HYDROGEN ENERGY, ELSEVIER, AMSTERDAM, NL, Bd. 24, Nr. 8, (1999-08-01), Seiten 771-777, DOI: 10.1016/S0360-3199(98)00128-1 ist es bekannt, Methan in die Elemente aufzuspalten und den Kohlenstoff langfristig der Atmosphäre zu entziehen. Schließlich ist die Herstellung von synthetischen Kohlenwasserstoffen aus Kohlenstoffdioxid und Wasserstoff aus der Schrift WO 2018/112654 A bekannt.

Die Fragestellung der Entfernung von emittiertem $CO_2$ aus der Luft (Carbon Dioxide Removal, CDR) gewinnt an Bedeutung. Mittlerweile gilt als gesichert, dass das Klimaziel der Begrenzung des Temperaturanstiegs auf 1,5°C beziehungsweise 2,0°C durch die Vermeidung von Emissionen allein nicht erreicht werden kann. Vielmehr erscheint eine kontinuierliche $CO_2$-Entnahme aus der Atmosphäre erforderlich zu sein, die bis zum Jahr 2100 100 GT bis 1000 GT $CO_2$ umfassen soll gemäß dem IPCC Special Report "Global warming of 1.5°C" des Intergovernmental Panel on Climate Change (IPCC), Oktober 2018. Es existieren nur wenige Verfahren, mit denen das bereits freigesetzte $CO_2$ aus der Atmosphäre abgeschieden wird.

Diese lassen sich im Wesentlichen in vier Gruppen unterteilen:

i) Verbrennung von Biomasse und Lagerung von $CO_2$: BECCS (bioenergy with carbon capture and storage),
ii) Entnahme von $CO_2$ aus der Umgebungsluft durch physikalische und/oder chemische Sorptionsprozesse, insbesondere durch Chemisorption mittels Aminen, und anschließendes Verpressen - DACCS (direct air carbon capture and storage),
iii) Anreicherung von Kohlenstoff auf Äckern durch Hinzufügen von Biokohle,
iv) Verfahren, die die Fähigkeit der Böden beziehungsweise der Gewässer erhöhen, $CO_2$ aufzunehmen. So werden auf Landflächen zerkleinerte Mineralien verteilt, die die chemische Bindung von $CO_2$ aus der Luft unterstützten. Ferner wurde eine Ozean-Alkalisierung vorgeschlagen, wobei zermahlene Mineralien den pH-Wert des Wassers senken und damit die $CO_2$-Speicherung im Meer begünstigen.

In den BECCS- und DACCS-Verfahren wird zugrunde gelegt, dass $CO_2$ aufgefangen und unterirdisch sequest-riert wird. Beim BECCS-Verfahren wird die Biomasse als Brennstoff benutzt und biogenes $CO_2$ aus dem Abgas entfernt. Beim DACCS-Verfahren wird $CO_2$ mittels Sorptionsverfahren, also chemischen beziehungsweise physikalischen Prozessen, aus der Umgebungsluft entnommen. Anschließend muss das gasförmige $CO_2$ zu räumlich entfernten Lagerstätten transportiert und geologisch gelagert werden.

Bei den BECCS-, DACCS- und allgemein CCS-Verfahren wird $CO_2$ abgetrennt und als solches gespeichert, wobei bekannte Nachteile durch das Verpressen und die Einlagerung von Gasen vorliegen.

Beim DACCS-Verfahren wird Umgebungsluft mithilfe von Ventilatoren durch Adsorptionsmaterial gefördert, wobei $CO_2$ an der Oberfläche des Adsorptionsmaterials physikalisch und/oder chemisch gebunden wird. Nachdem ein Sättigungsgleichgewicht erreicht wird, wird in einem zweiten Schritt $CO_2$ durch Erwärmen desorbiert, so dass das Adsorptionsmaterial regeneriert wird. Üblicherweise werden in derartigen Anlagen mindestens zwei Apparate eingesetzt, die phasenversetzt, also abwechselnd, arbeiten, wobei in einem der Apparate die Adsorption stattfindet, während im zweiten Apparat die Regenerierung durchgeführt wird. Ein solcher Wechsel wird auch als Swing-Verfahren bezeichnet. Dieses Verfahren ist dadurch gekennzeichnet, dass erhebliche Luftmengen gefiltert werden müssen, was durch die geringe Konzentration von $CO_2$ in der Luft (ca. 400 ppm) bedingt ist.

Es wird gasförmiges $CO_2$ produziert, das zum Beispiel geologisch dauerhaft eingelagert werden muss. Es ist also auch hier eine Lagerung in Gasform notwendig.

Zum Betrieb einer DACCS-Anlage ist mit Blick auf die $CO_2$-Bilanz weiterhin die Verfügbarkeit einer emissionsfreien Energie zur Erzeugung von Wärme, die zur Desorption benötigt wird, erforderlich, ebenso wie die Eignung geologischer Formationen zur Einlagerung des gasförmigen $CO_2$.

Unter den weiteren Möglichkeiten zur Bindung von $CO_2$ ist das Deponieren von Biomasse in grüner Form, beispielsweise durch Kompostieren, nicht zielführend, um eine dauerhafte $CO_2$-Entnahme aus der Atmosphäre zu gewährleisten, da es bei Abbauprozessen von Biomasse durch aerobe Prozesse wieder zur Bildung von $CO_2$ kommt. Weiterhin müssen beispielsweise bei der Einlagerung von festem Holz in Form von Holzstämmen neben $CO_2$-Emissionen lange Wachstumszeiten der Biomasse von mehreren Jahren bis Jahrzehnten in Kauf genommen werden.

Auch wird diskutiert, Biomasse zu pyrolysieren und den dabei entstehenden Kohlenstoff, der auch als Biochar bezeichnet wird, zu deponieren bzw. in der Landwirtschaft zu nutzen. Auch dabei kann es zu erneuten $CO_2$-Emissionen kommen, da Kohlenstoff hier der oxidierenden Wirkung von Luftsauerstoff ausgesetzt sein kann. Weiterhin bleibt in dieser Form die in der Biomasse befindliche Energie ungenutzt. Die Biomasse besteht jedoch zu einem erheblichen Teil aus Wasserstoff, der bei energetischer Nutzung keine Emissionen von $CO_2$ hervorrufen würde. Dieses Potenzial geht bei der Deponierung beziehungsweise landschaftlichen Nutzung von pyrolysierter Biomasse verloren.

Darüber hinaus kann die Ausbringung von pH-verändernden Chemikalien wie Mineralstoffen auf Landflächen beziehungsweise zur Ozean-Alkalisierung noch nicht bekannte Umwelteffekte zur Folge haben.

Eine Aufgabe der Erfindung besteht darin, $CO_2$ aus der Atmosphäre und/oder einem Abgas abzutrennen und eine Reemission verlässlich zu verhindern. Weiterhin soll die dazu benötigte Menge an Energie reduziert werden beziehungsweise zumindest ein Teil der im Verfahren umgewandelten Energie nutzbar sein.

Offenbarung der Erfindung

Es wird ein Verfahren zur Entfernung und Immobilisierung von Kohlenstoffdioxid aus der Atmosphäre und/oder einem Abgas vorgeschlagen, umfassend die Schritte:

a) Umwandeln des Kohlenstoffdioxids aus der Atmosphäre und/oder einem Abgas in Biomasse mittels Photosynthese, bevorzugt auf Agrarflächen, insbesondere in einem Gewächshaus,
b) Durchführen einer Biogasreaktion, bei der die in Schritt a) erzeugte Biomasse zu Biogas, enthaltend Methan und Kohlenstoffdioxid, umgesetzt wird, insbesondere in einem Biogasreaktor,
c) Abtrennen des Methans aus dem erhaltenen Biogas,
d) Spalten des Methans in Kohlenstoff und Wasserstoff, wobei der Kohlenstoff als Feststoff gewonnen wird,
e) Auffangen des erhaltenen Kohlenstoffs,
f) Deponieren des erhaltenen Kohlenstoffs und
g) Umsetzen von Kohlenstoffdioxid aus dem Biogas mit dem in Schritt d) gewonnenen Wasserstoff zu einem Kohlenwasserstoffgemisch.

Das erfindungsgemäße Verfahren umfasst drei biologische beziehungsweise chemische Reaktionen um $CO_2$ aus der Luft zu entfernen. Dies sind die Photosynthese in Schritt a), die Biogasreaktion, insbesondere eine anaerobe Vergärung, in Schritt b) und das Spalten des Methans, insbesondere eine Pyrolyse von Methan, in Schritt d).

In Schritt a) findet die Photosynthese statt. Mit der Photosynthese werden aus Ausgangsstoffen mit einem geringen Energieniveau, nämlich $CO_2$ und Wasser, unter Einwirkung von Licht Substanzen mit höherem Energieniveau gebildet. In der Natur führt die Photosynthese zur Bildung von Biomasse sowie ihren Folgeprodukten wie Bioabfällen und auch Wirtschaftsdünger zum Beispiel in Form von Mist und Gülle. Die Reaktion der **Photosynthese** bewirkt Wachstum von Pflanzen und Algen in der Natur. Auch für die industrielle Produktion von Biomasse in Gewächshäusern oder Algen-Bioreaktoren wird die Photosynthese genutzt.

Schritt a) zur Herstellung der Biomasse kann in einem Agrarprozess, also auf einer landwirtschaftlich betriebenen Fläche, und/oder in einem Gewächshaus stattfinden.

Als Biomasse werden insbesondere Substanzen und deren Gemische definiert, die mit der Photosynthese erzeugt werden. Diese werden auch als primäre Biomasse bezeichnet. Unter der Biomasse werden weiterhin auch Stoffe und deren Gemische verstanden, die in Folge der Nutzung der primären Biomasse entstanden sind und biogenen Charakter beibehalten haben. Die Biomasse kann insbesondere Stroh, Forstreste, Wirtschaftsdünger, Nahrungsmittelreste und/oder kommunale Abfälle enthalten.

In industriellen Anwendungen ist es vorteilhaft, die Biomasse-Produktion durch Anpassung der Reaktionsparameter zu optimieren wie der Konzentration von $CO_2$ in der Umgebungsluft. Die Effizienz der Photosynthese steigt pflanzenabhängig mit erhöhter Konzentration von $CO_2$. Bei $C_4$-Pflanzen wie Mais, Zuckerrohr oder Hirse wird das maximale Wachstum, also die maximale Effizienz der Photosynthese, bereits bei den heutigen atmosphärischen Bedingungen bezüglich der $CO_2$-Konzentration erreicht. Bei $C_3$-Pflanzen wie Weizen, Roggen, Gerste, Kartoffeln, Sojabohnen und Bäumen steigen die Wachstumsraten mit steigender $CO_2$-Konzentration weiter an. Bevorzugt werden in Schritt a) $C_3$-Pflanzen zur Umwandlung von $CO_2$ mittels Biosynthese eingesetzt. Eine maximale Effizienz der Photosynthese bei $C_3$-Pflanzen wird bei mehr als 1000 ppm $CO_2$ erreicht. Insgesamt können durch Erhöhung der $CO_2$-Konzentration in der Umgebungsluft bei optimalen Rahmenbedingungen in Bezug auf Temperatur, Bewässerung und Nährstoffversorgung, insbesondere mit Stickstoff, bis zu 40% höhere Erträge in der Biomasseproduktion, insbesondere in Gewächshäusern, erreicht werden. Durch Einsatz von schnellwachsenden $C_3$-Pflanzen kann die Ausbeute weiter gesteigert werden. Eine erhöhte Konzentration von $CO_2$ erhöht auch die Wachstumsrate von Algen.

Bei dem Abgas, dessen $CO_2$ in Schritt a) gegebenenfalls in Biomasse umgewandelt wird, kann es sich beispielsweise um ein Abgas handeln, das beim Verbrennen von fossilen Brennstoffen in einem Kraftwerk entsteht. Das Abgas kann beispielsweise auch ein Nebenprodukt sein, welches in einem industriellen Herstellungsprozess entsteht oder das Abgas kann ein Fördergas sein, welches bei der Förderung fossiler Energieträgern wie Kohle, Erdöl oder Erdgas anfällt. Ein Beispiel für ein Fördergas ist Grubengas. Den größten Anteil an den anfallenden Abgasen trägt die Herstellung von elektrischem Strom aus fossilen Brennstoffen bei.

Ebenso bedeutend als Quelle für klimarelevante Abgase sind industrielle Prozesse. Ein Beispiel für einen industriellen Herstellungsprozess ist die Zementproduktion, bei der die Emissionen von $CO_2$ größtenteils auf das Kalzinierungsverfahren zurückzuführen sind. Bei der Herstellung von Eisen und Stahl sowie hierfür benötigten Hilfsstoffen enthalten die Produktionsabgase $CO_2$. Klimarelevante Abgase können beispielsweise auch in Raffinerien auftreten.

Diese Abgase, die normalerweise in die Atmosphäre abgegeben werden, können nach deren Entstehung aufgefangen und dem vorgeschlagenen Verfahren zugeführt werden.

Die industriellen Abgase enthalten üblicherweise neben den bereits erwähnten Kohlenstoffoxiden andere Stoffe, die bei der Behandlung des Abgases berücksichtigt werden müssen. Hierzu zählen unter anderem Methan, Wasserstoff, Wasserdampf und das Inertgas Stickstoff, wobei der Anteil an Stickstoff bis zu 97 Vol.-% betragen kann. Als weitere Abgasbestandteile können Verunreinigungen wie Schwefelwasserstoff, Quecksilber und/oder Schwermetale enthalten sein. Diese Verunreinigungen bedürfen einer Vorbehandlung, um vorgeschriebene Grenzwerte zu erreichen. Auch Sauerstoff kann in Konzentration von bis zu 6 Vol.-% im Abgas von Kraftwerken vorgefunden werden. Abgase, die während der Förderung von beispielsweise Erdgas entstehen, enthalten neben Methan $CO_2$ und Stickstoff mit relevanten Anteilen.

Bevorzugt wird das Abgas vor der Zuführung mit üblichen, bereits bekannten Verfahren von Staub, schwefelhaltigen Verbindungen und anderen Verunreinigungen wie Stickstoffoxiden, Chlorwasserstoff, Fluorwasserstoff, Quecksilber, anderen Metallen und anderen organischen oder anorganischen Substanzen gereinigt.

Das Abgas, das Schritt a) zugeführt wird, ist insbesondere ein Gemisch, das $CO_2$ umfasst. Des Weiteren kann das Abgas als weitere Komponenten mindestens ein Inertgas wie Stickstoff oder Argon umfassen und gegebenenfalls Wasserdampf.

Bevorzugt entstammt das in Schritt a) eingesetzte Abgas zumindest teilweise der Verwendung des in Schritt d) erzeugten Wasserstoffs beziehungsweise des in Schritt b) gebildeten Biogases, insbesondere des Methans. Gegebenenfalls wird das Abgas in einer Vorbehandlung entschwefelt, von den anderen Verunreinigungen gereinigt und/oder entstaubt. Weiter bevorzugt besteht das in Schritt a) eingesetzte Abgas ausschließlich aus Abgas, das innerhalb des Verfahrens zurückgeführt wird. Mehr bevorzugt entstammt das in Schritt a) eingesetzte Abgas ausschließlich dem in Schritt b) erzeugten Biogas, insbesondere der Verwendung des in Schritt d) erzeugten Wasserstoffs. Entsprechend kann das Abgas dem Biogas direkt oder indirekt, also nach anschließender Spaltung des Methans, entstammen.

Die Biogasreaktion in Schritt b) umfasst insbesondere eine anaerobe Vergärungsreaktion. Bei der anaeroben Vergärung wird die Biomasse zu Biogas, also einem Gemisch aus überwiegend Methan und Kohlenstoffdioxid, umgewandelt. Das in Schritt b) erzeugte Biogas enthält bevorzugt in Summe mindestens 90 Vol.-%, weiter bevorzugt mindestens 95 Vol.-%, Methan und $CO_2$, bezogen auf das gesamte Biogas.

Insbesondere wird die Biomasse unter Einwirkung verschiedener Mikroorganismen, wie Bakterien hydrolysiert. Während der Biogasreaktion liegt bevorzugt ein pH-Wert im Bereich von 6 bis 7, weiter bevorzugt von 6,5 bis 7 und insbesondere von 6,6 bis 6,7 vor. Die Temperatur während der Biogasreaktion ist bevorzugt konstant und liegt weiter bevorzugt in einem Bereich von 25°C bis 45°C, mehr bevorzugt von 30°C bis 40°C, insbesondere von 33°C bis 37°C, zum Beispiel bei 35°C. In der Biogasreaktion werden Substanzen wie Zucker, Aminosäuren und/oder Fette abgebaut. Die Hydrolyse wird bevorzugt von einer Fermentation oder Acetylierung gefolgt, wobei Säuren gebildet werden, die anschließend in Methan und Kohlenstoffdioxid umgewandelt werden. Die beschriebenen Reaktionsbedingungen stellen ein Optimum für methanbildende Bakterien dar.

Als Biomasse werden in Schritt b) bevorzugt Pflanzen, insbesondere $C_3$-Pflanzen, Bioabfälle, Wirtschaftsdünger, wie Mist und/Gülle, und/oder kommunale Abfälle eingesetzt. Die in Schritt a) erzeugte Biomasse kann direkt in Schritt b) eingesetzt werden. So können in Schritt a) erzeugte Pflanzen direkt der Biogasreaktion in Schritt b) zugeführt werden. Alternativ kann die in Schritt a) erzeugte Biomasse zumindest teilweise zuerst einer Benutzung oder Umwandlung ausgesetzt sein, so dass diese in Schritt b) als Bioabfälle, kommunale Abfälle oder Dünger vorliegt.

Die Biogasreaktion wird bevorzugt in einem geschlossenen Behälter ausgeführt, der auch als Fermenter bezeichnet wird. Vor Durchführung der Biogasreaktion kann die Biomasse zerkleinert und gegebenenfalls sortiert werden. Die Biomasse kann dem Fermenter kontinuierlich zugeführt werden. Die Verweilzeit der Biomasse in dem Fermenter beträgt bevorzugt mehr als einen Tag. Das produzierte Biogas sammelt sich insbesondere in einem oberen Bereich des Fermenters über einer Flüssig- und Feststoffphase an. Das Biogas enthält bevorzugt mindestens 40 Vol.-%, weiter bevorzugt 50 Vol.-% bis 75 Vol.-%, insbesondere 62 Vol.-% bis 75 Vol.-% Methan, bezogen auf das gesamte Biogas. Weiterhin enthält das Biogas $CO_2$ und gegebenenfalls Wasserdampf, Schwefelwasserstoff und/oder Ammoniak.

Die kontrollierte Vergärung, die Biogasreaktion, insbesondere in einem geschlossenen Behälter, bietet den Vorteil, dass Emissionen, zum Beispiel in Form von Säuren, die Grundwasser kontaminieren können oder Methan-Emissionen, die unkontrolliert in die Atmosphäre gelangen und so zu Klimaveränderungen beitragen, vermindert werden.

Das erzeugte Biogas enthält $CO_2$, das in einer Aufbereitung des Biogases, insbesondere einer $CO_2$-Abtrennvorrichtung, abgetrennt und der Photosynthese erneut zugeführt werden kann. Schritt c) kann entsprechend auch als Aufarbeitung des Biogases bezeichnet werden.

Zur Abtrennung des Kohlenstoffdioxids können zum Beispiel Polyimid-Hohlfaser-Membranen verwendet werden.

In einem Schritt g) wird $CO_2$ aus dem Biogas mit in Schritt d) erzeugtem Wasserstoff zu einem Kohlenwasserstoffgemisch umgesetzt.

Bevorzugt wird das $CO_2$ aus dem Biogas nach der Abtrennung von Methan in Schritt c) der Umsetzung mit dem in Schritt d) erzeugten Wasserstoff zugeführt. Das bei der Umsetzung des $CO_2$ mit dem Wasserstoff erhaltene Kohlenwasserstoffgemisch enthält insbesondere Kerosin, Benzin und/oder Wachse.

Zur Umsetzung des $CO_2$ aus dem erhaltenen Biogas zu einem Kohlenwasserstoffgemisch, kann das $CO_2$ insbesondere einer Fischer-Tropsch-Vorrichtung zugeführt werden.

Alternativ zu einer Fischer-Tropsch-Vorrichtung kann das $CO_2$ auch mit dem in Schritt d) erzeugten Wasserstoff zu Methan und Wasser umgewandelt werden. Dies kann zum Beispiel in einer katalytischen Sabatier-Reaktion oder einem biochemischen Prozess, bei dem methanbildende Mikroorganismen eingesetzt werden, erfolgen. Geeignete methanbildende Mikroorganismen sind zum Beispiel *Methanobacterium, Methanospirillium hungatii* oder *Methanosaeta.*

Neben der Umsetzung von $CO_2$ mit dem in Schritt d) gewonnenen Wasserstoff zu einem Kohlenwasserstoffgemisch kann ein Teil des $CO_2$ aus dem erhaltenen Biogas nach der Abtrennung von Methan gemäß Schritt c) in Schritt a) zurückgeführt werden. Ferner kann ein Teil des $CO_2$ aus dem erhaltenen Biogas nach der Abtrennung von Methan, gemäß Schritt c), verflüssigt werden.

Um die Ausbeute an festem Kohlenstoff zu erhöhen, ist es weiterhin besonders bevorzugt, die Schritt g) gewonnenen Kohlenwasserstoffe zumindest teilweise in die Reaktion zur Spaltung des Methans in Schritt d) einzuleiten. In diesem Fall wird nicht nur das Methan in Kohlenstoff und Wasserstoff gespalten, sondern auch die in dem Kohlenwasserstoffgemisch enthaltenen Kohlenwasserstoffe.

Bevorzugt erfolgt das Abtrennen des Methans in Schritt c) physikalisch, insbesondere mittels Kondensation, Adsorption und/oder einem Membranverfahren. Das Abtrennen des Methans aus dem erhaltenen Biogas in Schritt c) wird bevorzugt kontinuierlich durchgeführt.

Für das Abtrennen des Methans wird insbesondere Druckwechseladsorption, Temperatur-Vakuum-Adsorption, chemische Adsorption, Membrantrennung, Druckgaswäscheverfahren und/oder Wechselkonzentration-Adsorption eingesetzt.

Nach dem Abtrennen in Schritt c) enthält der Stoffstrom des abgetrennten Methans bevorzugt mehr als 95 Vol.-% Methan und weiter bevorzugt nicht mehr als 1 Vol.-% $CO_2$.

In Schritt d), der auch als Pyrolyse bezeichnet werden kann, wird mindestens ein Teil des in Schritt b) gebildeten Methans und, sofern die in Schritt g) gewonnenen Kohlenwasserstoffe ebenfalls in Schritt d) zurückgeführt werden, die Kohlenwasserstoffe in die Elemente Wasserstoff und Kohlenstoff gespalten. Die Reaktionen, die in Schritt d) stattfinden, können mit folgenden Reaktionsgleichungen dargestellt werden:

$$CH_4 \rightarrow 2H_2 + C$$

$$CH_3(CH_2)_{nC}H_3 \rightarrow (n+3)H_2 + (n+2)C$$

Diese Reaktionen sind endotherm, das heißt es muss Energie dem Reaktionssystem zugeführt werden, damit die Reaktion abläuft.

Bevorzugt erfolgt das Spalten des Methans und gegebenenfalls der Kohlenwasserstoffe in Schritt d) mittels eines Pyrolyseverfahrens und insbesondere bei einer Temperatur von mindestens 800°C, mehr bevorzugt mindestens 1000°C, insbesondere mehr als 1200°C, weiter bevorzugt mehr als 1400°C.

Die für die Durchführung der vorstehend genannten Reaktion benötigte Energie wird bevorzugt in Form von Strom aus erneuerbaren Quellen wie Wind, Biogas und/oder Photovoltaik bereitgestellt, der insbesondere derzeit im Stromnetz nicht abgenommen werden kann. Es wird insbesondere kein fossiler Brennstoff hierzu verwendet. Alternativ kann ein Teil des Biogases bzw. des gewonnenen Wasserstoffs für das Bereitstellen der für die Spaltung notwendigen Energie verwendet werden.

Das Spalten des Methans und gegebenenfalls der Kohlenwasserstoffe kann mittels Thermopyrolyse durchgeführt werden, bei der die notwendige Energie durch direktes oder indirektes Erwärmen bereitgestellt wird. Bezüglich der direkten thermischen Spaltung sind Verfahren bekannt, bei denen Methan und/oder Kohlenwasserstoffe durch eine Säule aus flüssigem Metall in Form von Blasen strömen, sich dabei erwärmen und gespalten werden. Kohlenstoff sammelt sich an der Oberfläche der Metallsäule, Wasserstoff entweicht und wird der weiteren Verarbeitung zugeführt. Ferner ist ein thermisches Verfahren bekannt, in dem die Spaltung in einem aus Kohlenstoffgranulat bestehenden Wanderbett vorgenommen wird.

Weiterhin kann das Spalten des Methans und gegebenenfalls der Kohlenwasserstoffe unter Verwendung eines Lichtbogens durchgeführt werden, wobei die Energie durch elektrisches Entladen in der Gasphase zugeführt wird. Ein solches Verfahren ist dem Fachmann als Kvaerner-Verfahren bekannt. Das Spalten des Methans und gegebenenfalls der Kohlenwasserstoffe kann entsprechend in einem Plasma-Wasserstoffgenerator durchgeführt werden. Das Plasma wird beispielsweise durch Einstrahlen von elektromagnetischen Wellen erzeugt. Die Plasma-Spaltung des Methans und gegebenenfalls der Kohlenwasserstoffe findet bei Temperaturen von bis zu 2000°C und bei lokal hoher Energiedichte statt.

Das Spalten des Methans und gegebenenfalls der Kohlenwasserstoffe kann alternativ in Anwesenheit von mindestens einem Katalysator bei erhöhter Temperatur, insbesondere in einem Bereich von 800°C bis 1000°C, vorgenommen werden. Als Katalysator kann mindestens ein Metall, insbesondere ausgewählt aus der Gruppe bestehend Fe, Ni und Cr, und/oder Aktivkohle eingesetzt werden.

Bei der Pyrolyse entsteht eine Gasphase, die Wasserstoff als gasförmiges Produkt enthält, und Kohlenstoff, insbesondere elementarer Kohlenstoff, als Feststoff. Durch diese Reaktion werden der Energieträger Methan und gegebenenfalls die Kohlenwasserstoffe in zwei Stoffe gespalten. Der Wasserstoff kann bezüglich der Einwirkung aufs Klima unbedenklich energetisch genutzt werden, da beim Verbrennen von Wasserstoff mit Luft kein Treibhausgas, sondern Wasser beziehungsweise Wasserdampf entsteht. Würde alternativ die Verbrennung von Ruß zur Erzeugung der entsprechenden Energie durchgeführt werden, würden beispielsweise 3,67 kg klimaschädliches $CO_2$ bei der Verbrennung von 1 kg Ruß entstehen.

Die Gasphase nach der Pyrolyse umfasst bevorzugt Wasserstoff, ein Inertgas, Methan und gegebenenfalls Kohlenwasserstoffe. Die Gasphase kann zumindest teilweise einer Trennvorrichtung, insbesondere einer Membran, zugeführt werden, wobei Wasserstoff und ein Restgas entstehen.

Bevorzugt ist Schritt d) ein Filter nachgeschaltet, in dem der Kohlenstoff vom Wasserstoff getrennt wird. Die Umsetzung des Methans und gegebenenfalls der Kohlenwasserstoffe in Wasserstoff und Kohlenstoff erfolgt bevorzugt mit einer Ausbeute von ca. 96 bis 97%.

Bevorzugt wird nach dem Spalten des Methans und gegebenenfalls der Kohlenwasserstoffe in Schritt d) der Wasserstoff von dem Restgas, also weiteren gasförmigen Komponenten, abgetrennt, insbesondere mittels eines Membranverfahrens.

Der Wasserstoff kann stofflich oder energetisch genutzt werden und dazu einem Kraftwerk oder einem Transport- und Distributionssystem zugeführt werden. Das nach der Abtrennung des Wasserstoffs von der Gasphase verbleibende Restgas wird bevorzugt dem Kraftwerk zugeführt. Das Kraftwerk umfasst bevorzugt einen Gasmotor zu Erzeugung von Strom und Wärme. Die im Kraftwerkt erzeugte Wärme kann zumindest teilweise zur Erzeugung von Kälte verwendet werden. Auch bereits die Gasphase, also insbesondere ohne Abtrennung des Wasserstoffs, kann dem Kraftwerk zugeführt werden.

Der im Kraftwerk erzeugte Strom wird bevorzugt zum Betrieb des Reaktors zum Spalten von Methan und gegebenenfalls der Kohlenwasserstoffe eingesetzt und/oder netzstabilisierend ins öffentliche Stromnetz eingespeist. Ferner entstehen im Kraftwerk Wärme und Abgas, die zur Erzeugung der Biomasse in Schritt a), insbesondere in einem Gewächshaus, eingesetzt werden können.

In einer bevorzugten Ausführungsform wird ein Teil des Wasserstoffs in den Reaktor zum Spalten des Methans und gegebenenfalls der Kohlenwasserstoffe zurückgeführt.

Bevorzugt wird zumindest ein Teil des bei der Spaltung erzeugten Wasserstoffs als Ausgangsstoff in der chemischen Industrie, als Energieträger für die Erzeugung von elektrischem Strom und/oder Wärme, wobei die Wärme gegebenenfalls zumindest teilweise zur Erzeugung von Kälte verwendet werden kann, oder als Kraftstoff für Fahrzeuge verwendet. Zur Energieerzeugung kann der Wasserstoff mit Verbrennungsluft gemischt und in einer Gasturbine verbrannt werden. Die Gasturbine kann einen Stromgenerator antreiben, mit dem elektrischer Strom erzeugt wird. Die Abgase aus der Verbrennung können über einen Dampferzeuger zur Erzeugung von Prozessdampf eingesetzt werden.

Denkbar ist es auch, den Wasserstoff über eine Brennstoffzelle in elektrischen Strom umzusetzen oder den Wasserstoff für andere Zwecke, beispielsweise zum Betanken von wasserstoffbetriebenen Fahrzeugen oder zum Heizen einzusetzen.

Bevorzugt wird zumindest ein Teil des in Schritt d) erzeugten Wasserstoffs bzw. des in Schritt b) erzeugten Biogases, als Energiequelle für das Spalten des Methans und gegebenenfalls der Kohlenwasserstoffe in Schritt d) benutzt werden. Bevorzugt wird für das Spalten gemäß Schritt d) benötigte Energie zumindest teilweise durch Verwendung des in Schritt d) erzeugten Wasserstoffs und gegebenenfalls des Restgases bereitgestellt und/oder aus erneuerbaren Quellen erzeugt. Alternativ oder zusätzlich kann auch das in Schritt c) abgetrennte Methan zur Erzeugung von Strom und/oder Wärme eingesetzt werden. So können zum Beispiel 30 bis 50 Vol.-% des erzeugten Biogases, insbesondere in Form von Methan und/oder Wasserstoff, zur Erzeugung von Strom und/oder Wärme eingesetzt werden.

Bevorzugt wird der in Schritt d) erzeugte Wasserstoff zumindest teilweise als Ausgangsstoff für Synthesen in der chemischen Industrie, als Energieträger für die Erzeugung von elektrischem Strom, Wärme, gegebenenfalls Kälte und/oder als Kraftstoff für Fahrzeuge verwendet.

Bevorzugt wird der Wasserstoff nach dem Spalten des Methans und gegebenenfalls der Kohlenwasserstoffe gemäß Schritt d) vom Kohlenstoff abgetrennt, insbesondere mittels Filtration. Der abgetrennte Wasserstoff wird zusam-

men mit dem $CO_2$ aus dem Biogas dem Schritt g) zugeführt, um Kohlenwasserstoffe, insbesondere Methan zu erzeugen. Hierdurch kann die Ausbeute von festem Kohlenstoff erheblich erhöht werden.

Der in Schritt d) als Feststoff gewonnene Kohlenstoff wird insbesondere nicht als Energieträger benutzt. Der Kohlenstoff wird bevorzugt gesammelt, zu einer Deponiestätte transportiert und dauerhaft eingelagert bzw. endgelagert. Bevorzugt wird der in Schritt d) erhaltene Kohlenstoff vollständig deponiert. Weiter bevorzugt wird der in Schritt d) erhaltene Kohlenstoff von der Gasphase, die den Wasserstoff enthält, abgetrennt.

Weiter bevorzugt wird der in Schritt d) erhaltene Kohlenstoff vor dem Deponieren in Schritt f) mit anderen Komponenten, insbesondere weiteren Feststoffen, so vermengt, dass eine energetische Nutzung nicht mehr möglich ist, um den Kohlenstoff endgültig zu immobilisieren und besonders sicher verwahren zu können. Als weitere Feststoffe können beispielsweise Sand, Lehm, Kies, Bauschutt, Schlacken, Steine, Abfälle, insbesondere aus Industriedemontagen, oder eine Kombination mehrerer dieser Materialien eingesetzt werden. Entsprechend wird der in Schritt d) erhaltene Kohlenstoff bevorzugt immobilisiert und durch das Vermischen des erhaltenen Kohlenstoffs mit einem weiteren Feststoff wird eine dauerhafte Immobilisierung sichergestellt. Das gebildete Feststoffgemisch, das den erhaltenen Kohlenstoff mit mindestens einem weiteren Feststoff umfasst, wird insbesondere geologisch und dauerhaft in einer Kohlenstoffsenke, wie zum Beispiel einem Bergwerk, gelagert.

Bevorzugt wird der in Schritt d) als Feststoff erhaltene Kohlenstoff vor dem Deponieren gemäß Schritt f) mit mindestens einem weiteren Feststoff, insbesondere Sand und/oder Gestein vermischt.

Das Deponieren in Schritt f) erstreckt sich bevorzugt auf mindestens 30 Jahre, weiter bevorzugt mindestens 50 Jahre.

Eine Deponierung des Kohlenstoffs erfolgt bevorzugt unterirdisch, beispielsweise in alten Bergwerken, insbesondere Kalibergwerken oder Salzbergwerken. Der Kohlenstoff ist aber auch als Füllmaterial zum Verfüllen von Tagebauen, Abgrabungen, Kies-, Gips- oder Tongruben geeignet. Um geologische Schäden zu vermeiden sowie um den Naturschutzverpflichtungen nachzugehen, werden stillgelegte Förderstätten umfangreichen Sanierungs- und Rekultivierungsmaßnahmen unterzogen. Dabei werden die Hohlräume der Untertagebergwerke sowie die Gruben selbst mit mineralischem Material aufgefüllt. Als geeignetes Mittel kommen Bauschutt, Schlacken, Steine, Abfälle aus Industriedemontagen sowie andere industrielle Abfälle, die eine ausreichende Festigkeit aufweisen, zum Einsatz. Bei alleiniger Verwendung von Kohlenstoff müsste dieser für das Verfüllen zuerst verpresst werden. Als vorteilhaft erweist sich das Vermengen des pulverförmigen Kohlenstoffs mit Mineralien oder Abfällen, da Kohlenstoff in die poröse Struktur der Mineralien eindringen und dort dauerhaft fixiert werden kann.

Unter dem Deponieren in Schritt f) des erfindungsgemäßen Verfahrens kann auch die Verwendung des als Feststoff entstehenden Kohlenstoffs in der Bauindustrie, insbesondere dem Straßenbau, und/oder in der Landwirtschaft verstanden werden.

Die stoffliche Verwendung von als Feststoff entstehendem Kohlenstoff in industriellen Produkten wie Ummantelungen von Elektrokabeln, Dämmstoffen, Isolierungen, unterirdischen Bauten etc. kann auch als Deponieren klassifiziert werden, solange sichergestellt wird, dass sich die Lebensdauer der Anwendungen auf mindestens 30 Jahre, mehr bevorzugt mindestens 50 Jahre erstreckt. Hierbei wird der Kohlenstoff in fester Form in der Regel in das für die Produkte eingesetzte Material eingemischt.

Bevorzugt umfasst das Deponieren ausschließlich das geologische, insbesondere unterirdische, Deponieren, zum Beispiel in Bergwerken.

Bevorzugt werden mindestens die Schritte a) bis e) und Schritt g) in einem Modul durchgeführt. Unter einem Modul wird eine räumlich zusammenhängende Einheit verstanden. Weiter bevorzugt werden in dem Modul Kohlenwasserstoffe hergestellt, wobei $CO_2$ und das Restgas eingesetzt werden.

Weiterhin werden bevorzugt mehrere Module an jeweils unterschiedlichen Orten installiert.

Bevorzugt werden in mindestens zwei Modulen, also in mindestens zwei jeweils räumlich zusammenhängenden Einheiten, jeweils mindestens die Schritte a) bis e) und g) durchgeführt und weiter bevorzugt sind die mindestens zwei Module mit einer zentralen Leitstelle verbunden.

Bevorzugt wird das Vermischen des erhaltenen Kohlenstoffs mit einem weiteren Feststoff außerhalb oder unabhängig von den Modulen durchgeführt. Dabei entstammt der erhaltene Kohlenstoff aus mindestens einem Modul. Das Mischen kann mit der zentralen Leitstelle, insbesondere datentechnisch, verbunden sein.

Vorteile der Erfindung

Mit dem beschriebenen Verfahren wird das aus der Atmosphäre und/oder dem Abgas "extrahierte" $CO_2$ der Atmosphäre entzogen. Eine dauerhafte Immobilisierung des Kohlenstoffs ist möglich und somit wird ein Reinigungseffekt für die Atmosphäre erzielt.

Im Vergleich zur Verbrennung dieses Kohlenstoffs kann also eine geologische Speicherung und entsprechende Bindung von 3,67 kg $CO_2$ äquivalent bei Deponierung von 1 kg Kohlenstoff erzielt werden.

Die Kombination der Photosynthese, der anaeroben Vergärung, der Pyrolyse und der Deponierung, also der Schritte a), b), d) und f) ermöglicht somit die Reduktion von $CO_2$ in der Atmosphäre. Im Gegensatz zu oben beschriebenen bekannten anderen Verfahren zur Abtrennung von $CO_2$ aus der Atmosphäre entstehen mit dem erfindungsgemäßen Verfahren Wasserstoff und gegebenenfalls biogenes $CO_2$, die wirtschaftlich genutzt werden können.

Die Umwandlung von $CO_2$ in Kohlenstoff in fester Form vereinfacht in erheblichem Maße die Sequestrierung, da ein Feststoff und kein Gas immobilisiert wird. Das Finden einer geeigneten Kohlenstoff-Deponie erscheint um vielfaches leichter als das Auffinden geeigneter Lagerkapazitäten für gasförmiges $CO_2$, wie sie für herkömmliche CCS-Verfahren erforderlich sind.

Ferner eliminiert das Deponieren von Kohlenstoff in fester Form die Risiken der Wiederemission. Geologische Risiken, die mit Verpressen des gasförmigen Kohlenstoffdioxids unter hohem Druck einhergehen, entfallen.

Die Umsetzung des im Biogas enthaltenen $CO_2$ mit dem in der Spaltung gewonnenen Wasserstoff zu Kohlenwasserstoffen inklusive Methan wirkt sich positiv auf die Ausbeute an festem Kohlenstoff aus. Die Menge an Kohlenstoff kann im Vergleich zu einem Verfahren ohne Einleitung der Kohlenwasserstoffe in die Spaltung bis zur Verdoppelung erhöht werden. Dies vergrößert konsequenterweise den angestrebten Reinigungseffekt der Atmosphäre von klimaschädlichem $CO_2$.

Anhand der nachstehenden Zeichnungen (Fig. 1 - 4), der Bezugszeichenliste und der Patentansprüche sowie der Beispiele wird die Erfindung eingehender beschrieben.

Es zeigen:

Figur 1      ein Schema des erfindungsgemäßen Verfahrens in einer ersten Ausführungsform,

Figur 2      ein Schema des Erfindungsgemäßen Verfahrens in einer zweiten Ausführungsform

Figur 3      ein Schema einer Verknüpfung mehrerer Module zur Durchführung des erfindungsgemäßen Verfahrens und

Figur 4      einen Ausschnitt eines Moduls zur Durchführung von mehreren Schritten des erfindungsgemäßen Verfahrens,

Figur 5      Prozessschema des ersten in Beispiel 4 durchgeführten Verfahrens,

Figur 6      Prozessschema des zweiten in Beispiel 4 durchgeführten Verfahrens.

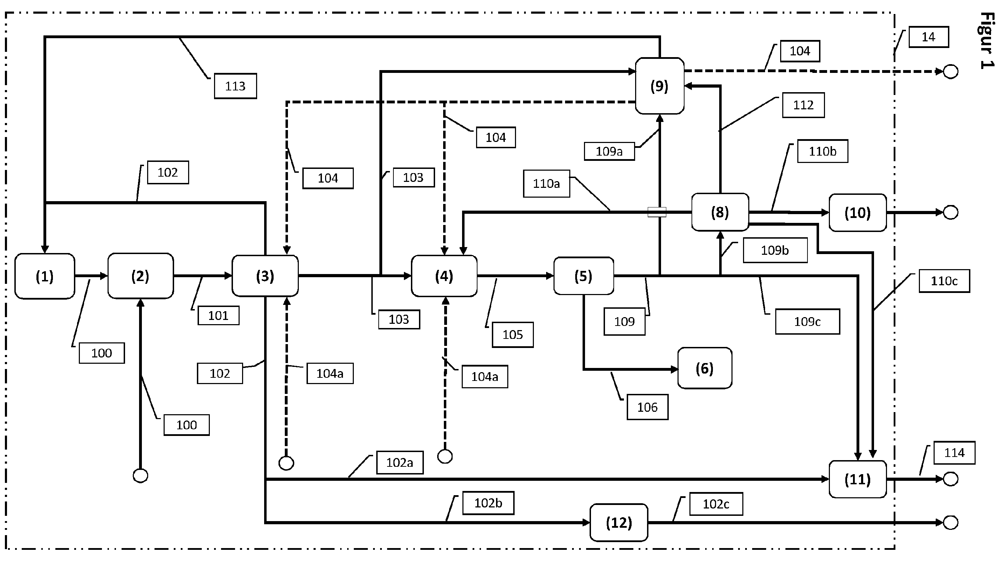

Figur 1 zeigt ein Schema des erfindungsgemäßen Verfahrens, wobei die durchgezogenen Pfeile Materialströme und die gestrichelte Pfeile Energieströme anzeigen. Die Kreise stellen Anknüpfungspunkte mit der Umgebung des Systems dar und die Strichzweipunktlinie markiert die Grenzen des betrachteten Moduls 14.

In Schritt a) wird Biomasse 100 mittels Photosynthese aus $CO_2$ 102 erzeugt. Dies geschieht beispielsweise durch Pflanzenwachstum auf einem Feld oder alternativ oder zusätzlich durch den Anbau von Pflanzen in einem Gewächshaus 1. Durch gezielte Auswahl der Pflanzen kann ein schnelles Wachstum der Biomasse 100 begünstigt und die Eignung zur Herstellung von Biogas 101 optimiert werden.

Für die Steigerung des Pflanzenwachstums kann die Luft im Gewächshaus 1 mit $CO_2$ 102 angereichert und erwärmt werden. Das $CO_2$ 102 kann separat und/oder als Teil eines Abgases 113 dem Gewächshaus 1 zugeführt werden. Dabei wird das $CO_2$ 102 bevorzugt aus weiteren Schritten des Verfahrens rückgeführt, insbesondere aus einer Aufarbeitung 3 von im Verfahren hergestelltem Biogas 101 und als Teil des Abgases 113 aus einem Blockheizkraftwerk (BHKW) 9.

Die Biomasse 100 wird in Schritt b) des Verfahrens, insbesondere mit einer anaeroben bakteriellen Reaktion, zu Biogas 101, das Methan 103 und $CO_2$ 102 enthält, vergärt. Die Reaktion wird insbesondere kontinuierlich in einem Mischbehälter, der einen Biogasreaktor 2 darstellt, durchgeführt.

In Schritt c) des Verfahrens wird das Biogas 101 einer Aufarbeitung 3 zugeführt, wobei Methan 103 in Form eines Methan-reichen Stroms, von einem $CO_2$ 102 enthaltenden Strom abgetrennt wird. Das $CO_2$ 102, zum Beispiel der gesamte $CO_2$ 102 enthaltende Strom, kann in das Gewächshaus 1 zurückgeführt werden, um die Luft im Gewächshaus 1 mit $CO_2$ 102 anzureichern. Alternativ oder ergänzend zur Rückführung des $CO_2$ 102 von der Aufarbeitung 3 in das Gewächshaus 1 wird mindestens ein Teil 102a des $CO_2$ 102 zu einer Fischer-Tropsch und/oder Methanisierungs-Vorrichtung 11 geleitet.

Ein weiterer Teil 102b des $CO_2$ kann einer $CO_2$-Verflüssigung 12 zugeführt werden, der dann flüssiges $CO_2$ 102c entnommen werden kann.

In Schritt d) des Verfahrens wird das Methan 103 von der Aufarbeitung 3 einem Reaktor 4 zugeführt. Dort wird es stark erhitzt. In dem Reaktor 4 wird Methan 103 in seine Bestandteile Kohlenstoff als Feststoff 106 und Wasserstoff 110 gespalten. Alle genannten Reaktionstypen, also die Reaktion mittels Pyrolyse, beispielsweise Thermopyrolyse, Lichtbogen-Pyrolyse, Plasma-Pyrolyse oder katalytischer Pyrolyse, können hier zur Anwendung kommen.

Die Umsetzung des Methans 103 ist nicht vollständig und andere Kohlenwasserstoffe fallen in geringen Mengen ebenfalls an. Am Ausgang des Reaktors 4 entsteht ein Produktgasgemisch 105, das gasförmiges Methan 103, Wasserstoff 110 und andere Kohlenwasserstoffe 114 sowie Kohlenstoff als Feststoff 106 enthält.

Zum Betrieb des Reaktors 4 wird bevorzugt Energie wie elektrischer Strom 104a aus erneuerbaren Quellen eingesetzt, insbesondere aus Windenergie und Photovoltaik. Der elektrische Strom 104 kann alternativ oder zusätzlich in der BHKW-Anlage 9 erzeugt werden.

Dem Reaktor 4 ist ein Filter 5 nachgeschaltet, in dem die Trennung des Feststoffs 106 von einer Gasphase 109 erfolgt, die Wasserstoff 110 enthält.

Zumindest ein Teil 109a der Gasphase 109 kann gegebenenfalls zwischengelagert und als Brenn- beziehungsweise Treibstoff der integrierten BHKW-Anlage 9 zugeführt werden. Weiterhin kann der in der BHKW-Anlage erzeugte elektrische Strom 104 netzstabilisierend ins öffentliche Stromnetz eingespeist werden.

Alternativ kann der Wasserstoff 110 in einer $H_2$-Trennvorrichtung 8, beispielsweise einer Wechseldruckadsorptionsanlage oder mittels Membranen, von in der Gasphase 109 vorhandenem Restgas 112 separiert werden. Hierzu wird mindestens ein Teil 109b der Gasphase 109 der Membran 8 zugeführt. Ein Teil des Wasserstoffs 110a kann in den Reaktor 4 zurückgeführt werden, ein weiterer Teil als Produkt 110b weiterverwertet werden. Hierzu kann der als Produkt 110b weiterverwertete Wasserstoff zunächst einer $H_2$-Kommissionierung 10 zugeführt werden und beispielsweise komprimiert und in Gasflaschen abgefüllt oder in eine Pipeline zum Weitertransport eingeleitet werden.

Erfindungsgemäß wird mindestens ein Teil 109c der Gasphase 109 oder alternativ des Wasserstoffs 110c der Fischer-Tropsch und/oder Methanisierungs-Vorrichtung 11 zugeführt. Hier kann der Wasserstoff aus der Gasphase 109 oder der Wasserstoff 110 zusammen mit $CO_2$ 102, das der Aufarbeitung 3 entstammt, in ein Kohlenwasserstoffgemisch 114 umgewandelt werden. Das Kohlenwasserstoffgemisch 114 enthält bevorzugt Kerosin, Benzin, Wachse und Mischungen daraus.

Alternativ zu der hier genannten Fischer-Tropsch-Vorrichtung 11 kann auch jedes andere, dem Fachmann bekannte Verfahren zur Erzeugung von Kohlenwasserstoffen, beispielsweise eine katalytische Sabatier-Reaktion oder ein biochemischer Prozess, bei dem methanbildende Mikroorganismen eingesetzt werden, eingesetzt werden. In der Fischer-Tropsch und/oder Methanisierungs-Vorrichtung 11 oder bei dem alternativ eingesetzten Verfahren können längerkettige Kohlenwasserstoffe, beispielsweise Kerosin, Benzin und/oder Wachse hergestellt werden, oder auch Methan oder kürzerkettige Kohlenwasserstoffe wie Ethan, Propan oder Butan sowie Mischungen daraus.

Das nach der Abtrennung aus der Gasphase 109 verwendete und als Brennbeziehungsweise Treibstoff dienende Restgas 112 kann gegebenenfalls zwischengelagert und der BHKW-Anlage 9 zugeführt werden. Als Alternative zu einer externen Stromquelle kann der in der BHKW-Anlage 9 erzeugte elektrische Strom 104 für die Spaltung des Methans 103 im Reaktor 4 genutzt werden. Weiterhin werden die in der BHKW-Anlage 9 erzeugten Abgase 113, insbesondere mit der dort erzeugten Wärme 115 bevorzugt dem Gewächshaus 1 zugeführt.

Das in Figur 1 dargestellte Verfahren kann zu einem Modul 14 örtlich zusammengefasst werden. Ein Modul 14 kann der örtlichen Versorgung mit Strom 104, Wasserstoff 110 und Wärme 115 sowie der Herstellung von Kohlenwasserstoffen 114 dienen.

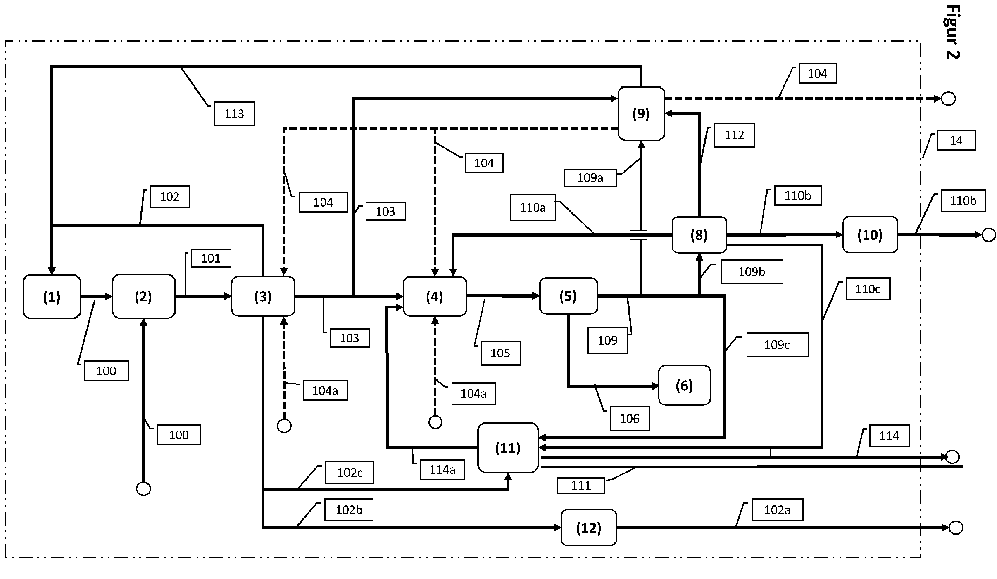

Figur 2 zeigt eine alternative Ausführungsform des erfindungsgemäßen Verfahrens, die sich von der in Figur 1 gezeigten Ausführungsform insbesondere durch die Einleitung der in der Fischer-Tropsch und/oder Methanisierungs-Vorrichtung 11 erzeugten Kohlenwasserstoffe und/oder des Methans in den Reaktor 4 unterscheidet.

Durch die Einleitung zumindest eines Teils 114a der in der Fischer-Tropsch und/oder Methanisierungs-Vorrichtung erzeugten Kohlenwasserstoffe und/oder des Methans in den Reaktor 4 kann die Ausbeute an festem Kohlenstoff 106 deutlich erhöht werden.

In diesem Fall wird im Reaktor nicht nur das Methan 103 zu Kohlenstoff 106 und Wasserstoff 110 umgesetzt, sondern auch die in den Reaktor eingeleiteten Kohlenwasserstoffe und/oder Methans 114a, die in der Fischer-Tropsch-Reaktion erhalten wurden, werden in festen Kohlenstoff 106 und Wasserstoff 110 gespalten. Die durchgeführte Reaktion ist dabei die gleiche wie vorstehend für Figur 1 für die Spaltung des Methans beschrieben.

Zur Umsetzung des $CO_2$ zu Kohlenwasserstoffen 114, 114a in der Fischer-Tropsch und/oder Methanisierungs-Vorrichtung wird neben dem Teil 109c der Wasserstoff enthaltenden Gasphase 109 auch ein Teil 110a des in der $H_2$-Trennvorrichtung 8 abgetrennten Wasserstoffs in die Fischer-Tropsch und/oder Methanisierungs-Vorrichtung 11 eingeleitet. Alternativ zu der hier dargestellten Ausführungsform ist es auch möglich, nur den Teil 109c der Wasserstoff enthaltenden Gasphase 109 oder nur den Wasserstoff 110a in die Fischer-Tropsch und/oder Methanisierungs-Vorrichtung 11 einzuleiten.

Auch ist es möglich, wie hier dargestellt, einen Teil der in der Fischer-Tropsch und/oder Methanisierungs-Vorrichtung 11 gewonnenen Kohlenwasserstoffe und/oder des Methans 114 als Produkt zu entnehmen und nur einen Teil 114a in den Reaktor 4 einzuleiten. Bevorzugt ist es jedoch, um die maximale Ausbeute an festem Kohlenstoff 106 zu erhalten, die gesamten in der Fischer-Tropsch und/oder Methanisierungs-Vorrichtung 11 erzeugten Kohlenwasserstoffe und/oder das gesamte erzeugte Methan in den Reaktor 4 einzuleiten.

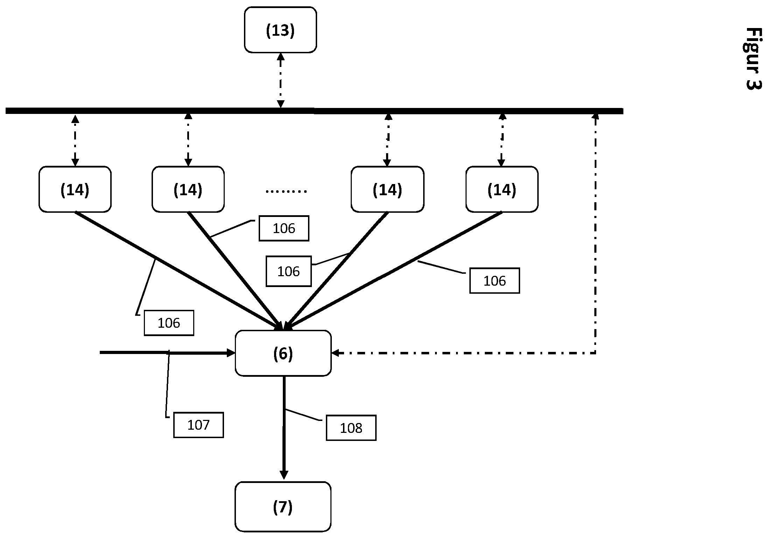

Figur 3 zeigt ein Schema einer Verknüpfung mehrerer Module 14, wobei die durchgezogenen Pfeile Materialströme und die Pfeile mit Strichpunkt-Linie den Datenfluss anzeigen. Die Module 14 verfügen über ein internes, also örtliches Transport- und Distributionssystem für die Verteilung des Wasserstoffs 110.

Die Module 14 sind datentechnisch miteinander verbunden, so dass eine optimierte Fahrweise hinsichtlich zum Beispiel des Produkt-Portfolios, der Auslastung oder der Distribution möglich ist. Die Optimierung der Steuerung der Module 14 erfolgt aus einer Leitstelle 13, an der Daten zentral erfasst, gespeichert und verarbeitet werden. Die Leitstelle 13 und die Module 14 kommunizieren miteinander über ein Datennetz wie dem Internet.

Der in den Modulen 14 anfallende Kohlenstoff als Feststoff 106 wird zu einer zentralen, ebenfalls mit der Leitstelle 13 verbundenen Mischvorrichtung 6 mit weiteren Feststoffen 107, die auch als Füllstoffe bezeichnet werden, wie Sand oder Schüttgut, zu einem Feststoffgemisch 108 verarbeitet. Das Feststoffgemisch 108 kann nun in einer Kohlenstoffsenke 7 wie zum Beispiel einer alten Grube geologisch und dauerhaft gelagert werden. Der Kohlenstoff als Feststoff 106 kann auch in anderen Anwendung eingesetzt werden, bei denen er dauerhaft immobilisiert wird, so dass er als Äquivalent für das aus der Luft entfernte $CO_2$ betrachtet werden kann. Alternativ kann der Kohlenstoff auch in Materialien, die zur Herstellung von Ummantelungen von Elektrokabeln, Isolierungen, Dämmstoffen oder zur Abdichtung unterirdischer Bauten genutzt werden, eingemischt werden.

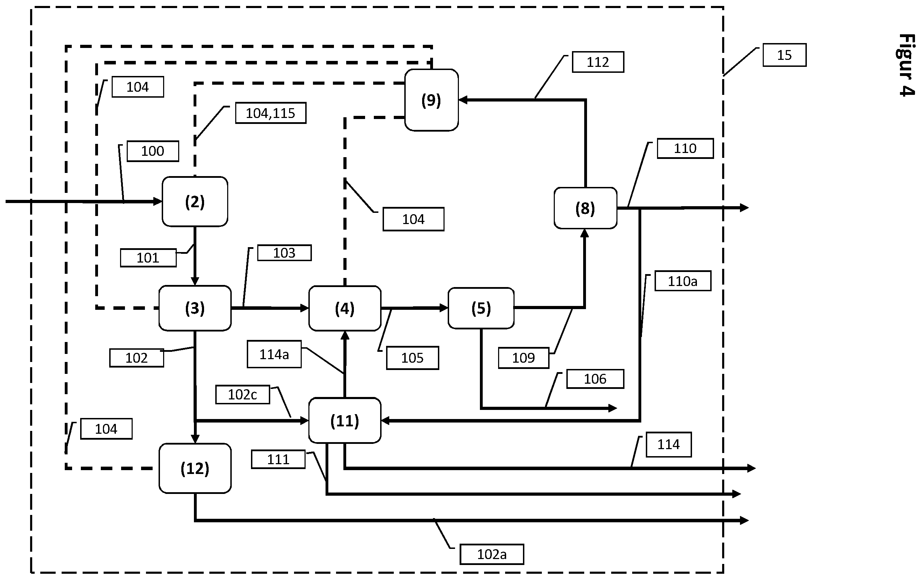

Figur 4 zeigt einen Ausschnitt eines Moduls 14 im Luft-Reinigungs-Modus. Die Bilanzgrenze 15 entspricht dem im Folgenden ausgeführten Beispiel 3. Es wird Biomasse 100 zum Biogasreaktor 2 zugeführt, der auch elektrischen Strom 104 und Wärme 115 von der BHKW-Anlage 9 erhält. Das dem Biogasreaktor 2 entnommene Biogas 101 wird in der Aufarbeitung 3 in (Bio-)Methan 103 und gasförmiges $CO_2$ 102 aufgetrennt. Das Methan 103 wird wiederum im Reaktor 4 zu einem Produktgemisch 105, also einem Kohlenstoff-GasGemisch, umgesetzt, wobei in dem Filter 5 Kohlenstoff als Feststoff 106, beispielsweise als Ruß, abgeführt wird.

Zumindest ein Teil 102a des gasförmigen $CO_2$ wird einer Fischer-Tropsch und/oder Methanisierungs-Vorrichtung 11 zur Erzeugung von Kohlenwasserstoffen und/oder Methan zugeführt. Ein Teil 114a der Kohlenwasserstoffe wird dem Reaktor 4 zur Spaltung in festen Kohlenstoff 106 und Wasserstoff 110 zugeführt. Nach Abtrennung aus dem in dem Reaktor erhaltenen Restgas wird zumindest ein Teil 110a des Wasserstoffs der Fischer-Tropsch und/oder Methanisierungs-Vorrichtung 11 zugeführt.

Sofern nicht die gesamten in der Fischer-Tropsch und/oder Methanisierungs-Vorrichtung 11 erzeugten Kohlenwasserstoffe 114a in den Reaktor 4 eingeleitet werden, kann ein Teil der Kohlenwasserstoffe 114 als Produkt entnommen werden.

Wenn nicht das gesamte $CO_2$ der Fischer-Tropsch und/oder Methanisierungs-Vorrichtung 11 zugeführt wird, kann das restliche $CO_2$ einer $CO_2$-Verflüssigung 12 zugeführt und dieser als flüssiges $CO_2$ 102c entnommen werden.

In der Fischer-Tropsch und/oder Methanisierungs-Reaktion entstehendes Wasser 111 wird kondensiert und aus der Vorrichtung entfernt.

Die im Filter 5 abgetrennte Gasphase 109 wird über die $H_2$-Trennvorrichtung 8 geführt, an der Wasserstoff 110 zur Verfügung steht. Das verbleibende Restgas 112 wird in der BHKW-Anlage 9 energetisch genutzt, wobei elektrischer Strom 104 innerhalb des Moduls 14 zur Verflüssigung des $CO_2$ 102 und elektrischer Strom 104 und Wärme 115 im Biogasreaktor 2 sowie elektrischer Strom 104 zur Spaltung des Methans 103 im Reaktor 4 eingesetzt werden.

Beispiele

*Ausführungsbeispiel 1: Entfernung von* $CO_2$ *aus der Luft mit Anbau von Mais*

Zur Quantifizierung der $CO_2$-Emissionen wird ein Teilsystem eines Moduls bilanziert, das einen Reaktor, einen Filter und eine $H_2$-Trennvorrichtung zur Wasserstoffabtrennung sowie eine BHKW-Anlage umfasst. Es wird weiterhin angenommen, dass keine externe Energie, also von außerhalb des Systems, zugeführt wird, was einem Setzen der Ströme 104a in Figur 1 gleich 0 entspricht.

In Abhängigkeit von der Zusammensetzung der verwendeten Biomasse ist das Methangemisch, also das erzeugte Biogas, nominell bereits unterschiedlich mit $CO_2$-Emissionen aus den vorgeschalteten Prozessschritten, insbesondere der Herstellung der Biomasse, belastet. In diesem Ausführungsbeispiel wird Mais als verwendete Biomasse betrachtet. Dem aus Mais gebildeten Methangemisch sind bereits folgende Emissionen aus der Herstellung des Mais zuzurechnen:

Tabelle 1:

| *Vorgang* | *Äquivalente* $CO_2$-Emissionen |
|---|---|
| Anbau | 17,7 g $CO_2$ eq./MJ |
| Verarbeitung (Vergärung in Schritt b)) | 4,3 g $CO_2$ eq./MJ |

(fortgesetzt)

| Vorgang | Äquivalente $CO_2$-Emissionen |
|---|---|
| Aufarbeitung des Biogases | 4,5 g $CO_2$ eq./MJ |
| Gesamt | 26,5 g $CO_2$ eq./MJ |

Das bilanzierte Teilsystem wird so betrieben, dass 60 Gew.-% des aus dem Mais erzeugten Methans stöchiometrisch zu Wasserstoff und Kohlenstoff umgewandelt werden. Die verbleibenden 40 Gew.-% des Methans werden zur Herstellung von elektrischem Strom verwendet, der in Figur 1 mit der Bezeichnung 104 der BHKW-Anlage 9 entnommen wird.

Die folgende Berechnung basiert auf einem Verbrauch von 100 kg Methan.

In der BHKW-Anlage wird Strom mit einem Wirkungsgrad von 36% erzeugt. Dem erzeugten Strom werden $CO_2$-Emissionen folgendermaßen zugerechnet:

$$40 \text{ kg } CH_4 \times 36\% \times 50 \text{ MJ/kg} \times 26,5 \text{ g } CO_2 \text{ eq./MJ} = 19,08 \text{ kg } CO_2 \text{ eq.}$$

Bei der Spaltung des Methans entstehen die beiden Produkte Kohlenstoff als Feststoff und Wasserstoff. Bei Einsatz von 60 kg Methan, dem wie folgt äquivalente $CO_2$-Emissionen zugerechnet werden, entstehen 45 kg Kohlenstoff und 15 kg Wasserstoff:

$$60 \text{kg} \times 50 \text{ MJ/kg} \times 26,5 \text{ g } CO_2 \text{ eq./MJ} = 79,5 \text{ kg } CO_2 \text{ eq.}$$

Der gebildete Kohlenstoff als Feststoff wird dauerhaft gebunden, da dieser deponiert wird. Dieser feste Kohlenstoff ist biogenen Ursprungs, so dass eine Gutschrift für nicht erfolgte Emissionen berücksichtigt wird, da diese Emissionen, die beispielsweise beim Verbrennen des festen Kohlenstoffs aufgetreten wären, der Atmosphäre nicht zugeführt werden. 1 kg gebundener fester Kohlenstoff entspricht einer Gutschrift von 3,67 kg $CO_2$ Aquivalent (eq.). Bei hier zu deponierenden 45 kg festen Kohlenstoffs entsteht also eine Gutschrift von 165,15 kg $CO_2$ eq.

Den hergestellten 15 kg Wasserstoff, die unter Berücksichtigung des Heizwertes von Wasserstoff von 120 MJ/kg $H_2$ einer nutzbaren Energie von 1800 MJ entsprechen, (15 kg x 120 MJ/kg $H_2$ = 1800 MJ) werden 79,5 kg $CO_2$ eq. (44,1 g $CO_2$ eq./MJ $H_2$) aus der Stromerzeugung in dem BHKW zugeordnet und ca. 1 g $CO_2$ eq./MJ aus dem Transport und dem Handling des festen Kohlenstoffs bei dauerhafter Lagerung zugerechnet. Die Treibhausgas-Quote für Wasserstoff beträgt dann 54,1 g $CO_2$ eq./MJ $H_2$.

Die Gutschrift aus der Einlagerung des festen Kohlenstoffs liegt wie oben ausgeführt bei - 165,15 kg $CO_2$ eq. pro 100 kg Methan (- 91,75 g $CO_2$ eq./MJ $H_2$). Wird diese Gutschrift bei der Bilanzierung des hergestellten Wasserstoffs berücksichtigt, so zeigt sich eine vorteilhafte $CO_2$-Bilanz des hergestellten Wasserstoffs von -46,65 g $CO_2$ eq/MJ $H_2$ (- 46,65 = 44,1 + 1,00 - 91,75).

*Ausführungsbeispiel 2: Entfernung von $CO_2$ aus der Atmosphäre unter Verwendung von Gülle*

Wird statt Mais Gülle zur Erzeugung des Methans im Biogasreaktor eingesetzt, so wird dem Wasserstoff ohne Berücksichtigung der Gutschrift für den eingelagerten festen Kohlenstoff eine $CO_2$-Emission von - 212,48 g $CO_2$ eq./MJ $H_2$ und mit Berücksichtigung der Gutschrift für die Deponierung des festen Kohlenstoffs von - 304,23 g $CO_2$ eq./MJ $H_2$ zugerechnet.

*Beispiel 3: Vergleich des erfindungsgemäßen Verfahrens mit bekannten OAC-Verfahren*

Für den Vergleich des erfindungsgemäßen Verfahrens, das auch als Luft-ReinigungsVerfahren bezeichnet werden kann, mit dem DAC (direct air capture)-Verfahren werden folgende Bedingungen festgelegt.

Für beide Verfahren wird die Entfernung von 1 t $CO_2$ aus der Umgebungsluft betrachtet.

Basierend auf S. Deutz, A. Bardow "Supplementary material: Life-cycle assessment for industrial direct air capture process based on temperature-vacuum swing adsorption", Nature Energy, 6, Seiten 203 bis 213 (2021), wird angenommen, dass ca. 0,7 kWh pro kg aus der Luft entferntes $CO_2$ für mechanische Arbeit wie den Betrieb von Ventilatoren und Pumpen und zusätzlich 3,3 kWh Wärme benötigt werden.

Bei der Verwendung von Wärmepumpen werden 1,3 kWh elektrischer Strom als Wärmeersatz notwendig, so dass sich ein gesamter Energiebedarf zuzüglich der Verflüssigung von 4,11 beziehungsweise 2,11 kWh/kg $CO_2$ ergibt.

Bezüglich der erfindungsgemäßen Luft-Reinigungs-Methode, die entsprechend in Figur 3 dargestellt ist, berücksichtigt die energetische Bilanz die Herstellung von Biogas aus Biomasse im Biogasreaktor 2, die Auftrennung des Biogases mittels Membranen in der Aufarbeitung 3 in $CO_2$ und Methan, die Spaltung von Methan mittels innerhalb des Verfahrens erzeugten Stroms im Reaktor 4, wobei der Strom mittels der BHKW-Anlage 9 erzeugt wurde, die Abscheidung des festen Kohlenstoffs im Filter 5, die mittels $CO_2$-Äquivalenten erfasst wurde, die Abtrennung des Wasserstoffs über die $H_2$-Trennvorrichtung 8 und die Verflüssigung von $CO_2$ in der $CO_2$-Verflüssigungsanlage 12.

Bei der Entfernung von 1 t $CO_2$ aus der Luft mit Hilfe des DAC-Verfahrens gemäß dem Stand der Technik werden 2,11 MWh elektrischen Stroms benötigt, während bei der Entfernung mittels des Luft-Reinigungs-Verfahrens 1 t $CO_2$ in Form von 157 kg Kohlenstoff und 423 kg $CO_2$ als Gas entfernt werden, wobei die für die beschriebenen Verfahrensschritte benötigte Energie ausschließlich aus Biogas gewonnen wird und zusätzlich 5 kg Wasserstoff und 1,1 MWh Wärme, wobei Temperaturen bis 500°C zur Verfügung stehen, produziert werden.

*Beispiel 4: Erhöhung der Kohlenstoff-Ausbeute mittels $CO_2$-Methanisierung*

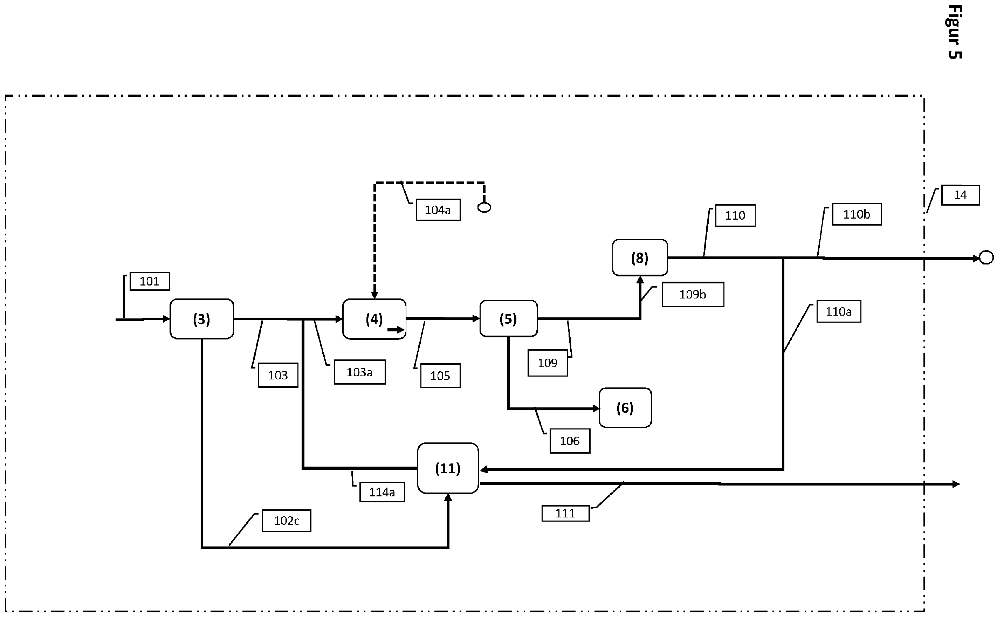

Das in diesem Beispiel verwendete System umfasst eine Biogas-Aufbereitung 3, einen Spaltungsreaktor 4, einen Kohlenstoff-Filter 5, eine $H_2$-Trennvorrichtung 8 und einen Methanisierung-Reaktor 11. Als Energiequelle, im Gegensatz zu den vorstehenden Beispielen, wird Strom aus erneuerbaren Quellen (EE-Strom) eingesetzt. Zur Vereinfachung wird auch angenommen, dass EE-Strom keine Treibhausgas-Belastung aufweist. Der Anteil des Methans im Biogas beträgt 58 Vol.-%. Die Ausbeuten der Reaktionen entsprechen der Stöchiometrie. Das Prozessschema ist in der Figur 5 dargestellt.

Zuerst erfolgt die Auftrennung des Biogases in Methan 103 und $CO_2$ 102c in der Biogas-Aufbereitung 3. Das Kohlenstoffdioxid 102c wird dem Methanisierungsreaktor 11 zugeführt, wo dieses mit Wasserstoff 110a, der in der $H_2$-Trennvorrichtung 8 gewonnen wird, zu Methan 114a umgesetzt wird. Das im Methanisierungsreaktor 11 erzeugte Methan 114a wird zusammen mit dem Methan 103 aus der Vorrichtung 3 dem Spaltungsreaktor 4 zugeführt, wo dieses zu festem Kohlenstoff 106 und gasförmigem Wasserstoff 110 umgesetzt wird. Kohlenstoff wird im Filter 5 abgetrennt, Wasserstoff wird in Vorrichtung 8 gereinigt und in zwei Ströme aufgeteilt. Ein erster Strom 110a wird in den Methanisierungsreaktor zurückgeführt und ein zweiter Strom 110b wird als Produkt komprimiert und verkauft.

Die Massenbilanz des Verfahrens ist in der Tabelle 2 dargestellt.

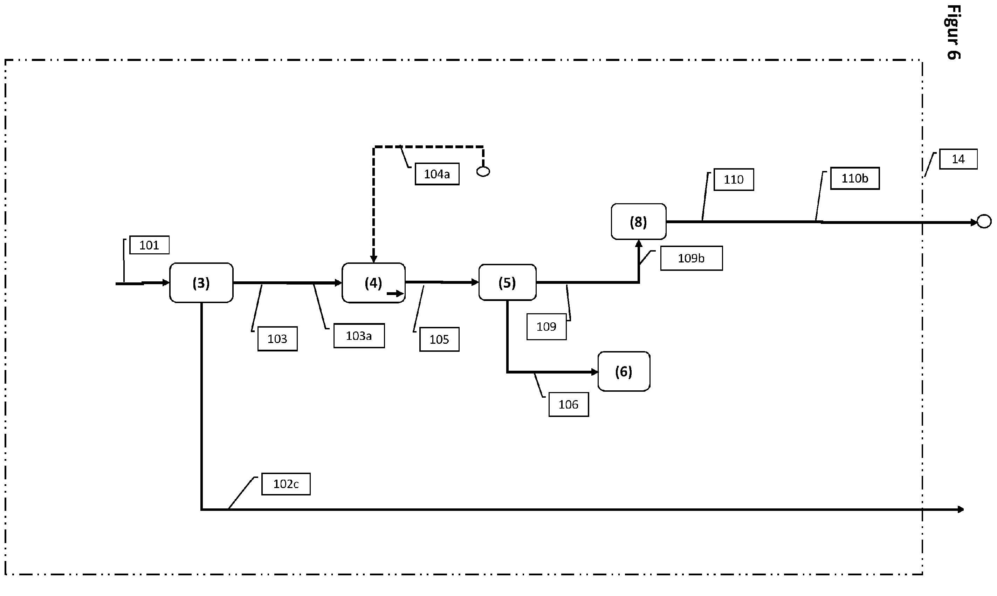

Alternativ kann auf die Umsetzung des im Biogas enthaltenen $CO_2$ im Methanisierungsprozess verzichtet werden. Dies ist beispielhaft in Figur 6 dargestellt. In diesem Fall wird $CO_2$ als Gas in die Atmosphäre emittiert oder verflüssigt und sequestriert. Die Massenbilanz des Prozesses ist in der Tabelle 3 abgebildet.

Durch die teilweise Rückführung des Wasserstoffs im Prozess gemäß Figur 5 wird die Ausbeute des festen Kohlenstoffs um etwa 70% erhöht. Die Ausbeute des Wasserstoffs, bedingt durch die Methanisierungsreaktion, wird entsprechend reduziert.

Tabelle 2:

| Strom Nr. | Massenstrom [kg/h] | $CH_4$ [kg/h] | $CO_2$ [kg/h] | $H_2$ [kg/h] | C [kg/h] | $H_2O$ [kg/h] |
|---|---|---|---|---|---|---|
| 101 | 332 | 112 | 220 | - | - | - |
| 102c | 220 | - | 220 | - | - | - |
| 103 | 112 | 112 | - | - | - | - |
| 114a | 80 | 80 | - | - | - | - |
| 103a | 192 | 192 | - | - | - | - |
| 105 | 192 | - | - | 48 | 144 | - |
| 106 | 144 | - | - | - | 144 | - |
| 109 | 48 | - | - | 48 | - | - |
| 110 | 48 | - | - | 48 | - | - |
| 110a | 40 | - | - | 40 | - | - |
| 110b | 8 | - | - | 8 | - | - |
| 111 | 180 | - | - | - | - | 180 |

Tabelle 3

| Strom Nr. | Massenstrom [kg/h] | CH$_4$ [kg/h] | CO$_2$ [kg/h] | H$_2$ [kg/h] | C [kg/h] | H$_2$O [kg/h] |
|---|---|---|---|---|---|---|
| 101 | 332 | 112 | 220 | - | - | - |
| 102c | 220 | - | 220 | - | - | - |
| 103 | 112 | 112 | - | - | - | - |
| 114a | - | - | - | - | - | - |
| 103a | 112 | 112 | - | - | - | - |
| 105 | 112 | - | - | 28 | 84 | - |
| 106 | 84 | - | - | - | 84 | - |
| 109 | 28 | - | - | 28 | - | - |
| 110 | 28 | - | - | 28 | - | - |
| 110a | - | - | - | - | - | - |
| 110b | 28 | - | - | 28 | - | - |
| 111 | - | - | - | - | - | - |

Bezugszeichenliste

| | |
|---|---|
| 1 | Gewächshaus |
| 2 | Biogasreaktor |
| 3 | Aufarbeitung von Biogas |
| 4 | Reaktor |
| 5 | Filter |
| 6 | Mischvorrichtung |
| 7 | Kohlenstoffsenke |
| 8 | H$_2$-Trennvorrichtung |
| 9 | Blockheizkraftwerk |
| 10 | H$_2$-Kommissionierung |
| 11 | Fischer-Tropsch und/oder Methanisierungs-Vorrichtung |
| 12 | CO$_2$-Verflüssigung |
| 13 | Leitstelle |
| 14 | Modul |
| 15 | Bilanzgrenze |
| 100 | Biomasse |
| 101 | Biogas |
| 102, 102a, 102b | CO$_2$ |
| 102c | flüssiges CO$_2$ |
| 103 | Methan |
| 103a | Methan- Kohlenwasserstoffgemisch |
| 104 | elektrischer Strom |
| 104a | externe Energie |
| 105 | Produktgemisch |
| 106 | Kohlenstoff als Feststoff |
| 107 | weiterer Feststoff |
| 108 | Feststoffgemisch |
| 109, 109a, 109b, 109c | Gasphase |
| 110, 110a, 110b, 110c | Wasserstoff |
| 111 | Wasser |
| 112 | Restgas |
| 113 | Abgas |
| 114, 114a, 114b | Kohlenwasserstoffgemisch |

115            Wärme

**Patentansprüche**

1. Verfahren zur Entfernung und Immobilisierung von Kohlenstoffdioxid (102) aus der Atmosphäre und/oder einem Abgas (113), umfassend die Schritte:

   a) Umwandeln des Kohlenstoffdioxids (102) aus der Atmosphäre und/oder einem Abgas (113) in Biomasse (100) mittels Photosynthese, bevorzugt auf Agrarflächen, insbesondere in einem Gewächshaus (1),
   b) Durchführen einer Biogasreaktion, bei der die in Schritt a) erzeugte Biomasse (100) zu Biogas (101), enthaltend Methan (103) und Kohlenstoffdioxid (102), umgesetzt wird, insbesondere in einem Biogasreaktor (2),
   c) Abtrennen des Methans (103) aus dem erhaltenen Biogas (101),
   d) Spalten des Methans (103) in Kohlenstoff (106) und Wasserstoff (110), wobei der Kohlenstoff (106) als Feststoff gewonnen wird,
   e) Auffangen des erhaltenen Kohlenstoffs (106),
   f) Deponieren des erhaltenen Kohlenstoffs (106) und
   g) Umsetzen von Kohlenstoffdioxid (102) aus dem Biogas mit dem in Schritt d) gewonnenen Wasserstoff (110) zu einem Kohlenwasserstoffgemisch (114).

2. Verfahren nach Anspruch 1, **dadurch gekennzeichnet, dass** das Kohlenstoffdioxid (102) aus dem erhaltenen Biogas (101) nach der Abtrennung von Methan (103) gemäß Schritt c) in Schritt a) zurückgeführt wird.

3. Verfahren nach mindestens einem der vorhergehenden Ansprüche, **dadurch gekennzeichnet, dass** zumindest ein Teil des in Schritt g) gewonnenen Kohlenwasserstoffgemischs (114) in Schritt d) eingeleitet und in Kohlenstoff (106) und Wasserstoff (110) gespalten wird.

4. Verfahren nach mindestens einem der vorhergehenden Ansprüche, **dadurch gekennzeichnet, dass** das in Schritt b) erzeugte Biogas (101) mindestens 40 Vol.-% Methan (103) enthält.

5. Verfahren nach mindestens einem der vorhergehenden Ansprüche, **dadurch gekennzeichnet, dass** die Biogasreaktion eine anaerobe Vergärungsreaktion umfasst.

6. Verfahren nach mindestens einem der vorhergehenden Ansprüche, **dadurch gekennzeichnet, dass** die Abtrennung des Methans (103) in Schritt c) physikalisch erfolgt, insbesondere mittels Kondensation, Adsorption und/oder einem Membranverfahren.

7. Verfahren nach mindestens einem der vorhergehenden Ansprüche, **dadurch gekennzeichnet, dass** das Spalten des Methans (103) in Schritt d) mittels eines Pyrolyseverfahrens und insbesondere bei einer Temperatur von mindestens 800°C erfolgt.

8. Verfahren nach mindestens einem der vorhergehenden Ansprüche, **dadurch gekennzeichnet, dass** der Wasserstoff (110) nach dem Spalten des Methans (103) gemäß Schritt d) vom Kohlenstoff (106) abgetrennt wird, insbesondere mittels Filtration.

9. Verfahren nach mindestens einem der vorhergehenden Ansprüche, **dadurch gekennzeichnet, dass** nach dem Spalten des Methans (103) in Schritt d) der Wasserstoff (110) von einem Restgas (112) abgetrennt wird, insbesondere mittels eines Membranverfahrens (8).

10. Verfahren nach mindestens einem der vorhergehenden Ansprüche, **dadurch gekennzeichnet, dass** der in Schritt d) erzeugte Wasserstoff (110) zumindest teilweise als Ausgangsstoff für Synthesen in der chemischen Industrie, als Energieträger für die Erzeugung von elektrischem Strom (104) und/oder Wärme (115) und/oder als Kraftstoff für Fahrzeuge verwendet wird.

11. Verfahren nach Anspruch 10, **dadurch gekennzeichnet, dass** für das Spalten gemäß Schritt d) benötigte Energie zumindest teilweise durch Verwendung des in Schritt d) erzeugten Wasserstoffs (110) und gegebenenfalls des Restgases (112) bereitgestellt wird und/oder aus erneuerbaren Quellen erzeugt wird.

12. Verfahren nach mindestens einem der vorhergehenden Ansprüche, **dadurch gekennzeichnet, dass** nach Spalten des Methans (103) in Schritt d) vom Wasserstoff (110) abgetrenntes Restgas (112) und/oder der in Schritt d) erhaltene Wasserstoff (110) und/oder in Schritt d) erhaltene Gasphase vor Abtrennung des Wasserstoffs einem Kraftwerk zugeführt werden und das in Schritt a) eingesetzte Abgas (113) zumindest teilweise dem Kraftwerk entstammt.

13. Verfahren nach mindestens einem der vorhergehenden Ansprüche, **dadurch gekennzeichnet, dass** mindestens die Schritte a) bis e) und gegebenenfalls Schritt g) in einem Modul (14) durchgeführt werden.

14. Verfahren nach Anspruch 13, **dadurch gekennzeichnet, dass** in mindestens zwei Modulen (14) jeweils mindestens die Schritte a) bis e) und gegebenenfalls Schritt g) durchgeführt werden, und die mindestens zwei Module (14) mit einer zentralen Leitstelle (13) verbunden sind.

15. Verfahren nach mindestens einem der vorhergehenden Ansprüche, **dadurch gekennzeichnet, dass** der in Schritt d) als Feststoff erhaltene Kohlenstoff (106) vor dem Deponieren gemäß Schritt f) mit mindestens einem weiteren Feststoff (107), insbesondere Sand und/oder Gestein, vermischt wird.

## Claims

1. Method for removing and immobilizing carbon dioxide (102) from the atmosphere and/or an exhaust gas (113), comprising the steps:

   a) Converting the carbon dioxide (102) from the atmosphere and/or an exhaust gas (113) into biomass (100) by means of photosynthesis, preferably on agricultural land, in particular in a greenhouse (1),
   b) carrying out a biogas reaction in which the biomass (100) produced in step a) is converted into biogas (101) containing methane (103) and carbon dioxide (102), in particular in a biogas reactor (2),
   c) separating the methane (103) from the biogas (101) obtained,
   d) splitting the methane (103) into carbon (106) and hydrogen (110), wherein the carbon (106) is obtained as a solid,
   e) collecting the carbon (106) obtained,
   f) depositing the carbon (106) obtained, and
   g) reacting carbon dioxide (102) from the biogas with the hydrogen (110) obtained in step d) to form a hydrocarbon mixture (114).

2. Method according to claim 1, **characterized in that** the carbon dioxide (102) from the biogas (101) obtained after separation of methane (103) according to step c) is returned to step a).

3. Method according to at least one of the preceding claims, **characterized in that** at least part of the hydrocarbon mixture (114) obtained in step g) is introduced into step d) and split into carbon (106) and hydrogen (110).

4. Method according to at least one of the preceding claims, **characterized in that** the biogas (101) produced in step b) contains at least 40 vol.-% methane (103).

5. Method according to at least one of the preceding claims, **characterized in that** the biogas reaction comprises an anaerobic fermentation reaction.

6. Method according to at least one of the preceding claims, **characterized in that** the separation of the methane (103) in step c) is carried out physically, in particular by means of condensation, adsorption and/or a membrane process.

7. Method according to at least one of the preceding claims, **characterized in that** the splitting of the methane (103) in step d) is carried out by means of a pyrolysis process and, in particular, at a temperature of at least 800°C.

8. Method according to at least one of the preceding claims, **characterized in that** the hydrogen (110) is separated from the carbon (106) after the methane (103) has been split in step d), in particular by filtration.

9. Method according to at least one of the preceding claims, **characterized in that** after splitting the methane (103) in step d), the hydrogen (110) is separated from a residual gas (112), in particular by means of a membrane process (8).

10. Method according to at least one of the preceding claims, **characterized in that** the hydrogen (110) produced in step d) is used at least partially as a starting material for syntheses in the chemical industry, as an energy carrier for the generation of electrical power (104) and/or heat (115) and/or as a fuel for vehicles.

11. Method according to claim 10, **characterized in that** energy required for the splitting according to step d) is provided at least in part by using the hydrogen (110) produced in step d) and, if applicable, the residual gas (112), and/or is generated from renewable sources.

12. Method according to at least one of the preceding claims, **characterized in that**, after splitting the methane (103) in step d), residual gas (112) separated from the hydrogen (110) and/or the hydrogen (110) obtained in step d) and/or gas phase obtained in step d) are fed to a power plant before the hydrogen is separated off, and the exhaust gas (113) used in step a) originates at least in part from the power plant.

13. Method according to at least one of the preceding claims, **characterized in that** at least steps a) to e) and, if applicable, step g) are carried out in a module (14).

14. Method according to claim 13, **characterized in that** at least the steps a) to e) and, if applicable, step g) are carried out in at least two modules (14), and the at least two modules (14) are connected to a central control center (13).

15. Method according to at least one of the preceding claims, **characterized in that** the carbon (106) obtained as a solid in step d) is mixed with at least one further solid (107), in particular sand and/or rock, before being deposited according to step f).


**Revendications**

1. Procédé pour enlever et immobiliser le dioxyde de carbone (102) de l'atmosphère et/ou d'un gaz d'échappement (113), comprenant les étapes suivantes :

   a) transformer le dioxyde de carbone (102) de l'atmosphère et/ou d'un gaz d'échappement (113) en biomasse (100) par photosynthèse, de préférence sur des terres agricoles, en particulier dans une serre (1),
   b) réalisation d'une réaction de biogaz dans laquelle la biomasse (100) produite à l'étape a) est transformée en biogaz (101) contenant du méthane (103) et du dioxyde de carbone (102), en particulier dans un réacteur à biogaz (2),
   c) séparation du méthane (103) du biogaz obtenu (101),
   d) décomposition du méthane (103) en carbone (106) et hydrogène (110), le carbone (106) étant récupéré sous forme solide,
   e) capture du carbone obtenu (106),
   f) mise en dépôt du carbone obtenu (106) et
   g) réaction du dioxyde de carbone (102) issu du biogaz avec l'hydrogène (110) obtenu à l'étape d) pour former un mélange d'hydrocarbures (114).

2. Procédé selon la revendication 1, **caractérisé en ce que** le dioxyde de carbone (102) est renvoyé dans l'étape a) après avoir été séparé du méthane (103) de la manière décrite à l'étape c) à partir du biogaz obtenu (101).

3. Procédé selon au moins une des revendications précédentes, **caractérisé en ce que** au moins une partie du mélange d'hydrocarbures (114) obtenu à l'étape g) est introduite à l'étape d) et décomposée en carbone (106) et hydrogène (110).

4. Procédé selon au moins une des revendications précédentes, **caractérisé en ce que** le biogaz (101) produit à l'étape b) contient au moins 40 % en volume de méthane (103).

5. Procédé selon au moins une des revendications précédentes, **caractérisé en ce que** la réaction du biogaz comprend une réaction de fermentation anaérobie.

6. Procédé selon au moins une des revendications précédentes, **caractérisé en ce que** la séparation du méthane (103) à l'étape c) est faite physiquement, surtout par condensation, adsorption et/ou un procédé à membrane.

7. Procédé selon au moins une des revendications précédentes, **caractérisé en ce que** la décomposition du méthane (103) à l'étape d) se fait par un procédé de pyrolyse, surtout à une température d'au moins 800 °C.

8. Procédé selon au moins une des revendications précédentes, **caractérisé en ce que** l'hydrogène (110) est séparé du carbone (106) après la décomposition du méthane (103) selon l'étape d), préférablement par filtration.

9. Procédé selon au moins une des revendications précédentes, **caractérisé en ce que** après la décomposition du méthane (103) à l'étape d), l'hydrogène (110) est séparé d'un gaz résiduel (112), préférablement avec un procédé à membrane (8).

10. Procédé selon au moins une des revendications précédentes, **caractérisé en ce que** l'hydrogène (110) produit à l'étape d) est au moins en partie utilisé comme matière première pour des synthèses dans l'industrie chimique, comme source d'énergie pour produire de l'électricité (104) et/ou de la chaleur (115) et/ou comme carburant pour des véhicules.

11. Procédé selon la revendication 10, **caractérisé en ce que** l'énergie nécessaire pour la séparation à l'étape d) est au moins en partie fournie par l'utilisation de l'hydrogène (110) produit à l'étape d) et, le cas échéant, du gaz résiduel (112), et/ou est produite à partir de sources renouvelables.

12. Procédé selon au moins l'une des revendications précédentes, **caractérisé en ce que** après la décomposition du méthane (103) dans l'étape d), le gaz résiduel séparé de l'hydrogène (110), (112) séparé de l'hydrogène (110) après la décomposition du méthane (103) à l'étape d) et/ou l'hydrogène (110) obtenu à l'étape d) et/ou la phase gazeuse obtenue à l'étape d) sont envoyés à une centrale électrique avant la séparation de l'hydrogène, et le gaz d'échappement (113) utilisé à l'étape a) provient au moins en partie de la centrale électrique.

13. Procédé selon au moins une des revendications précédentes, **caractérisé en ce que** au moins les étapes a) à e) et, si besoin, l'étape g) sont faites dans un module (14).

14. Procédé selon la revendication 13, où au moins les étapes a) à e) et, si besoin, l'étape g) sont faites dans au moins deux modules (14), et les au moins deux modules (14) sont connectés à un centre de contrôle (13).

15. Procédé selon au moins une des revendications précédentes, **caractérisé en ce que** le carbone (106) obtenu sous forme solide à l'étape d) est mélangé avec au moins un autre solide (107), en particulier du sable et/ou de la roche, avant d'être déposé conformément à l'étape f).

**Figur 1**

**Figur 2**

# Figur 3

**Figur 4**

Figur 5

Figur 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013112205 A1
- DE 102007037672 A1
- WO 2015044407 A1
- WO 2014170184 A1
- WO 2015185434 A1
- WO 2016005226 A1
- US 9095813 B
- US 201514815661 A
- US 8119091 B
- US 8728428 B
- US 9975100 B

- US 8871008 B
- US 9637393 B
- US 201715622883 A
- US 201715591324 A
- CA 2017051581
- GB 2470452 A
- CN 107058055 A
- US 2012276616 A
- US 2021371277 A
- WO 2018112654 A

**In der Beschreibung aufgeführte Nicht-Patentliteratur**

- Biokohle: Ein Weg zur dauerhaften Kohlenstoff-Sequestrierung. **KAMMAN et al.** Umweltbeobachtungs- und Klimafolgenforschungsstation Linden, Hessisches Landesamt für Umwelt und Geologie. Institut für Pflanzenökologie der Justus-Liebig-Universität Gießen, April 2010, 1-8
- **J. TEMPLE**. Klima-Ingenieure machen Ernst, CO2 aus der Luft waschen. *Technology Review*, June 2017, 49-52
- **WURZBACHER, J. A.** Development of a temperature-vacuum swing process for CO2 capture from ambient air. *ETH Zürich Research Collection*, 2015, https://doi.org/10.3929/ethz-a-010432423
- **GEBALD, C.** Development of amine-functionalized adsorbent for carbon dioxide capture from atmospheric air. *ETH Zürich Research Collection*, 2014, https://doi.org/10.3929/ethza-010171623

- **E. KATIFU**. Carbon dioxide absorption using fresh water algae and identifying potential uses of algal biomass. Faculty of Engineering and the Built Environment, University of the Witwatersrand, 2011
- Green path from fossil-based to hydrogen economy: An overview of carbon-neutral technologies. **MURADOV N Z et al.** INTERNATIONAL JOURNAL OF HYDROGEN ENERGY. ELSEVIER, 01 December 2008, vol. 33, 6804-6839
- Fossil fuel decarbonization technology for mitigating global warming. **STEINBERG M et al.** INTERNATIONAL JOURNAL OF HYDROGEN ENERGY. ELSEVIER, 01 August 1999, vol. 24, 771-777
- **S. DEUTZ** ; **A. BARDOW**. Supplementary material: Life-cycle assessment for industrial direct air capture process based on temperature-vacuum swing adsorption. *Nature Energy*, 2021, vol. 6, 203-213